(19) Europäisches Patentamt
European Patent Office
Office européen des brevets

(11) EP 2 287 610 A1

(12) EUROPEAN PATENT APPLICATION

(43) Date of publication:
**23.02.2011 Bulletin 2011/08**

(51) Int Cl.:
***G01N 33/543*** *(2006.01)*

(21) Application number: **09305773.5**

(22) Date of filing: **20.08.2009**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(71) Applicant: **Corning Incorporated**
**Corning NY 14831 (US)**

(72) Inventors:
- **Deshayes, Sophie**
**77515 Faremoutiers (FR)**
- **Van Zutphen, Steven**
**77780 Bourron Marlotte (FR)**

(74) Representative: **Le Roux, Martine et al**
**Cabinet Beau de Loménie**
**158, rue de l'Université**
**75340 Paris Cedex 07 (FR)**

Remarks:
Claims 16 - 21 are deemed to be abandoned due to non-payment of the claims fees (Rule 45(3) EPC).

# (54) Thiol-modified surface immobilization article and method

(57) A method and article to immobilize a protein, including, for example, combining the protein and a mixture comprised of an activated spacer compound having a maleimide group in a buffer solution, to form a maleimide-modified protein; and contacting the maleimide-linker-modified protein and a buffer swollen, thiol-modified, surface bound polymer to immobilize the maleimide-linker-modified protein on the polymer surface of the article. Also disclosed are articles having an immobilized protein thereon, and to methods of using the articles having an immobilized protein, as defined herein.

EP 2 287 610 A1

## Description

The entire disclosure of any publication, patent, or patent document mentioned herein is incorporated by reference.

## BACKGROUND

The disclosure relates generally to an article and method for processing proteins for use in, for example, immobilization, isolation, characterization, analysis, diagnosis, or like applications. The disclosure also relates to an article for use in processing proteins.

## SUMMARY

The disclosure provides an article and a method for processing a target protein. A target protein can be chemically modified to enhance the target protein's interaction with a thiol-modified surface of the article. The disclosure also provides an article having a thiol-modified surface for use in processing proteins, for example, immobilizing proteins and detecting ligands.

## BRIEF DESCRIPTION OF THE DRAWING(S)

In embodiments of the disclosure:

Fig. 1 shows an SH-maleimide coupling reaction;

Fig. 2 schematically shows the functionalization of a protein with a maleimide linker which is subsequently and specifically bound to a thiol-modified SH-surface.

Fig. 3 schematically illustrates a preparative sequence for a thiol surface.

Fig. 4 illustrates exemplary carbonic anhydrase (CAII) immobilization results on a thiol surface.

Fig. 5 illustrates furosemide binding results on immobilized carbonic anhydrase (CAII) at various concentrations of CAII.

Fig. 6 illustrates exemplary carbonic anhydrase (CAII) immobilization results on a thiol surface.

Fig. 7 illustrates furosemide binding results on carbonic anhydrase (CAII) that has been immobilized on a thiol surface.

Fig. 8 illustrates exemplary carbonic anhydrase (CAII) immobilization results on a thiol surface polymer corresponding to structural formula (**IVa**).

Fig. 9 illustrates exemplary furosemide binding results on immobilized carbonic anhydrase (CAII) on a thiol surface polymer corresponding to structural formula **(IVa).**

## DETAILED DESCRIPTION

Various embodiments of the disclosure will be described in detail with reference to drawings, if any. Reference to various embodiments does not limit the scope of the invention, which is limited only by the scope of the claims attached hereto. Additionally, any examples set forth in this specification are not limiting and merely set forth some of the many possible embodiments for the claimed invention.

### Definitions

"Assay," "assaying," or like terms refers to an analysis to determine, for example, the presence, absence, quantity, extent, kinetics, dynamics, or type of a biomolecule's or a cell's optical or bioimpedance response or like response or determination, upon stimulation with an exogenous stimuli, such as a ligand candidate compound. The analysis can include any of the above responses when, for example, a biomolecule interacts with the chemically modified surface of the article, or when a biomolecule associated with the chemically modified surface interacts with a ligand of the biomolecule.

"Attached" or like terms refers to any chemical interaction between two components or compounds. The type of chemical interaction that can be formed will vary depending upon the starting materials that are selected and reaction conditions. Examples of attachments described herein include, for example, covalent, electrostatic, ionic, hydrogen, or hydrophobic bonding. "Attach," "attachment," "adhere," "adhered," "adherent," "immobilized", or like terms can generally refer to immobilizing or fixing, for example, a surface modifier substance, a surface coating polymer, a compatibilizer, a cell, a ligand candidate compound, and like entities of the disclosure, to a surface, such as by physical absorption, chemical bonding, and like processes, or combinations thereof. A biosensor surface can be modified, such as having a

disclosed surface coating, an anchoring or tie material, a compatibilizer (e.g., fibronectin, collagen, lamin, gelatin, polylysine, etc.), or like modifications, and combinations thereof, that can promote, for example, receptivity of the biosensor surface towards particular molecular or cellular entities, such as protein binding and ligand detection.

"Contact" or "contacting" or like terms refer to, for example, an instance of exposure by an intimate physical encounter or touching of at least one substance to another substance.

"Target" or like terms refers to a cellular protein or cell-free biomolecule whose activation can mediate cell signaling or modulate cellular functions. A target can be, for example, a receptor, a phosphatase, a kinase, an enzyme, a DNA, an RNA, and like entities. A receptor can be, for example, a G protein-coupled receptor (GPCR), a receptor tyrosine kinase (RTK), a transporter, an ion channel, an integrin receptor, a sodium/proton exchanger, and like entities. A kinase can be, for example, protein kinase A, protein kinase C, mitogen-activated protein (MAP) kinases, an extracellular signal-regulated kinases, Src, Rho kinase, focal adhesion kinase, and like entities. An enzyme can be, for example, a membrane-bound adenylyl cyclase, a soluble adenylyl cyclase, a protease, and like entities.

"Screen," "screening," or like terms refers to, for example, a systematic survey of one or more compounds or drug candidates or biologicals (e.g., RNAi, antibody) to examine their pharmacological activities acting on a particular target, a cell type, or a cell system. Pharmacological or biological activity is an expression describing the beneficial or adverse effects of a drug on living matter. Aspects of the disclosure are particularly useful in biosensor-based high throughput screening (HTS) applications.

"Entrap," "entrapped," "entrapping," "entrapment," and like terms refer to, for example, an association of a protein with the functionalized surface of a substrate and at sufficient level for detection with, for example, optical instrumentation, but the protein is not necessarily irreversibly attached to the surface, see for example Pompe (Pompe, et. al, "Functional Films of Maleic Anhydride Copolymers under Physiological Conditions," Macromol. Biosci., 2005, 5, 890-895) and commonly owned and assigned EP Application No. 09290223.8, filed March 26, 2009, entitled "Immobilization Method for Protein Having Low Isoelectric Point."

"Immobilization," "immobilizing," "immobilize," "immobilized," and like terms refer to, for example, an association of a protein with the surface of the substrate which is substantially irreversible, such as not susceptible to easy removal from the surface by, for example, mild repeated rinsing or soaking in buffer.

"Protein" and like terms refers to the molecular target for entrapping or immobilization with the biosensor surface, and can include, for example, a peptide, a polypeptide, a glycoprotein, a lipoprotein, a receptor, a receptor component, an antibody, and like natural or synthetic molecules, or mixtures thereof. "Protein" can include an individual protein molecule, a protein molecule in admixture, in association, in complexation, or like relations with other molecular or biological entities, including for example, whole cells, or subunits or portions thereof.

"Biosensor" or like term refers to an article, that in combination with appropriate apparatus, can detect a desired analyte. A biosensor can combine a biological component with a physicochemical detector component. A biosensor can typically consist of three parts: a biological component or element (such as tissue, microorganism, pathogen, cells, cell component, a receptor, and like entities, or combinations thereof), a detector element (operating in a physicochemical way such as optical, piezoelectric, electrochemical, thermometric, magnetic, or like manner), and a transducer associated with both components. In embodiments, the biosensor can convert a molecular recognition, molecular interaction, molecular stimulation, or like event occurring in a surface bound cell component or cell, such as a protein or receptor, into a detectable and quantifiable signal. A biosensor as used herein can include liquid handling systems which are static, dynamic, or a combination thereof. In embodiments of the disclosure, one or more biosensor can be incorporated into a micro-article. Biosensors are useful tools and some exemplary uses and configurations are disclosed, for example, in PCT Application No. PCT/US2006/013539 (Pub. No. WO 2006/108183), published Dec. 10, 2006, to Fang, Y., et al., entitled "Label-Free Biosensors and Cells," and U.S. Patent No. 7,175,980. Biosensor-based cell assays having penetration depths, detection zones, or sensing volumes have been described, see for example, Fang, Y., et al. "Resonant waveguide grating biosensor for living cell sensing," Biophys. J., 91, 1925-1940 (2006). Microfluidic articles are also useful tools and some exemplary uses, configurations, and methods of manufacture are disclosed, for example, in U.S. Patent Nos. 6,677,131, and 7,007,709. U.S. Patent Publication 20070141231 and U.S. Patent No. 7,175,980, disclose a microplate assembly and method. The compositions, articles, and methods of the disclosure are particularly well suited for biosensors based on label independent detection (LID), such as for example an Epic® system or those based on surface plasmon resonance (SPR). The compositions, articles, and methods of the disclosure are also compatible with Dual Polarized Intereferometry (DPI), which is another type of LID sensor.

"EMA" generally refers to an ethylene-maleic anhydride (EMA) copolymer.

"dEMA" generally refers to "derivatized EMA," such as where residual maleic anhydride groups in the copolymer are converted or derivatized with a reagent such as an alkyl amine, a thiol-alkyl-amine, and like agents, or combinations thereof.

"Amine dEMA" refers to amine derivatized EMA copolymer. A propyl amine derivatized EMA copolymer is used as a surface coating on certain Epic® biosensor well-plate products commercially available from Corning, Inc.,

A "carboxy polymer surface," "surface bound carboxylate surface," or like terminology refers to a surface bound

polymer containing carboxylic acid functional groups (-C(=O)-OH), and carboxy -C(=O)- functional groups (A) which can attach the carboxy polymer to the substrate surface via the surface tie-layer groups or like functional groups, such as, hydroxy (-OH), amino (-NH-), and like functional groups. The polymer of the "carboxy polymer surface," can be, for example, an ethylene-maleic anhydride (EMA) polymer according to T.Pompe (*supra*). The polymer can be, for example, a polyacrylic acid polymer, a copolymer containing acrylic acid monomers, or like polymers.

"Activator," "activating agent," or like terms refer to a reagent that can react with a carboxylic acid, or like surface polymer substituent or target molecule substituent, to form a reactive intermediate. The reactive intermediate has increased reactivity towards nucleophiles such as amines, thiols, alcohols, or like functional groups compared to the acid. An "activated ester" is an ester having high reactivity towards nucleophiles, for example, a maleic anhydride mer, or the reaction product of a carboxylic acid group and an activating agent. The carboxylic acid groups can be, for example, on the carboxy surface, or on a protein, or other biomolecules.

"Hydrocarbon," "hydrocarbyl," "hydrocarbylene," "hydrocarbyloxy," and like terms refer to monovalent moieties such as -R, or divalent such as -R- moieties, and can include, for example, alkyl hydrocarbons, aromatic or aryl hydrocarbons, alkyl substituted aryl hydrocarbons, alkoxy substituted aryl hydrocarbons, heteroalkyl hydrocarbons, heteroaromatic or heteroaryl hydrocarbons, alkyl substituted heteroaryl hydrocarbons, alkoxy substituted heteroaryl hydrocarbons, and like hydrocarbon moieties, and as illustrated herein.

"Alkyl" includes linear alkyls, branched alkyls, cycloalkyls, and like structural dispositions.

"Substituted alkyl" or "optionally substituted alkyl "refers to an alkyl substituent, which can include, for example, linear alkyls, branched alkyls, or cycloalkyls, having from 1 to 4 optional substituents selected from, for example, hydroxyl (-OH), halogen, amino ($-NH_2$), nitro ($-NO_2$), alkyl, acyl (-C(=O)R), alkylsulfonyl ($-S(=O)_2R$), alkoxy (-OR), and like substituents, where R is a hydrocarbyl, aryl, Het, or like moieties, such as a monovalent alkyl or divalent alkylene having from 1 to about 10 carbon atoms. For example, a hydroxy substituted alkyl, can be a 2-hydroxy substituted propylene of the formula $-CH_2-CH(OH)-CH_2-$, an alkoxy substituted alkyl, can be a 2-methoxy substituted ethyl of the formula $-CH_2-CH_2-O-CH_3$, a 1-dialkylamino substituted ethyl of the formula $-CH(NR_2)-CH_3$, an oligo-(oxyalkylene), poly-(oxyalkylene), or poly-(alkylene oxide) substituted alkyl, can be, for example, of the partial formula $-(R-O)_x-$, where x can be, for example, from 1 to about 50, and from 1 to about 20, and like substituted oxyalkylene substituents, such as of the formula $-(CH_2-CHR^3-O)_x-$ where $R^3$ is a substituted or unsubstituted $(C_{1-8})$ alkyl, and x is an integer of from 1 to about 50. In embodiments, halo or halide includes fluoro, chloro, bromo, or iodo.

Alkyl, alkoxy, etc., include both straight and branched groups; but reference to an individual radical such as "propyl" embraces only the straight chain radical, a branched chain isomer such as "isopropyl" being specifically referred to.

The carbon atom content of various hydrocarbon-containing (i.e., hydrocarbyl) moieties can alternatively be indicated by a prefix designating a lower and upper number of carbon atoms in the moiety, i.e., the prefix $C_{i-j}$ indicates a moiety of the integer "i" to the integer "j" carbon atoms, inclusive. Thus, for example, $(C_1-C_7)$alkyl or $C_{1-7}$alkyl refers to alkyl of one to seven carbon atoms, inclusive, and hydrocarbyloxy such as $(C_1-C_8)$alkoxy or $C_{1-8}$alkoxy refers to alkyl of one to eight carbon atoms, inclusive.

Specifically, $C_{1-7}$alkyl can be, for example, methyl, ethyl, propyl, isopropyl, butyl, iso-butyl, sec-butyl, tert-butyl, pentyl, 3-pentyl, hexyl, or heptyl; $(C_{3-12})$cycloalkyl can be cyclopropyl, cyclobutyl, cyclopentyl, cyclohexyl, cycloheptyl, cyclooctyl, including bicyclic, tricyclic, or multi-cyclic substituents, and like substituents.

$(C_{1-8})$alkoxy can be methoxy, ethoxy, propoxy, isopropoxy, butoxy, iso-butoxy, sec-butoxy, pentoxy, 3-pentoxy, hexyloxy, 1-methylhexyloxy, heptyloxy, octyloxy, and like substituents.

$-(C_{2-7})$alkanoyl or $H-C(=O)(C_{1-6})$alkyl- can be acetyl, propanoyl, butanoyl, pentanoyl, 4-methylpentanoyl, hexanoyl, or heptanoyl.

Other conditions suitable for formation and modification of the compounds, oligomers, polymers, composites, or like products of the disclosure, from a variety of starting materials or intermediates, as disclosed and illustrated herein are known. For example, see Feiser and Feiser, "Reagents for Organic Synthesis", Vol. 1, et seq., 1967; March, J. "Advanced Organic Chemistry," John Wiley & Sons, 4th ed. 1992; House, H. O., "Modem Synthetic Reactions," 2nd ed., W. A. Benjamin, New York, 1972; and Larock, R. C., "Comprehensive Organic Transformations," 2nd ed., 1999, Wiley-VCH Publishers, New York. The starting materials employed in the preparative methods described herein are, for example, commercially available, have been reported in the scientific literature, or can be prepared from readily available starting materials using procedures known in the field. It may be desirable to optionally use a protecting group during all or portions of the disclosed or alternative preparative procedures. Such protecting groups and methods for their introduction and removal are known in the art. See Greene, T. W.; Wutz, P. G. M. "Protecting Groups In Organic Synthesis," 2nd ed., 1991, New York, John Wiley & Sons, Inc.

"Include," "includes," or like terms means encompassing but not limited to, that is, inclusive and not exclusive.

"About" modifying, for example, the quantity of an ingredient in a composition, concentrations, volumes, process temperature, process time, yields, flow rates, pressures, and like values, and ranges thereof, employed in describing the embodiments of the disclosure, refers to variation in the numerical quantity that can occur, for example: through

typical measuring and handling procedures used for making compounds, compositions, composites, concentrates or use formulations; through inadvertent error in these procedures; through differences in the manufacture, source, or purity of starting materials or ingredients used to carry out the methods; and like considerations. The term "about" also encompasses amounts that differ due to aging of a composition or formulation with a particular initial concentration or mixture, and amounts that differ due to mixing or processing a composition or formulation with a particular initial concentration or mixture. The claims appended hereto include equivalents of these "about" quantities.

"Consisting essentially of" in embodiments refers, for example, to a membrane polymer composition, to a method of making or using the membrane polymer, formulation, or composition, and articles, devices, or any apparatus of the disclosure, and can include the components or steps listed in the claim, plus other components or steps that do not materially affect the basic and novel properties of the compositions, articles, apparatus, or methods of making and use of the disclosure, such as particular reactants, particular additives or ingredients, a particular agents, a particular surface modifier or condition, or like structure, material, or process variable selected. Items that may materially affect the basic properties of the components or steps of the disclosure or that may impart undesirable characteristics to aspects of the disclosure include, for example, protein denaturation, or like functional disruption or changes to the protein's molecular structure or characteristics by chemical or physical means. Thus, conditions for protein handling and processing can be selected to approximate or match conditions the protein encounters in nature including, for example, avoiding adverse pH levels and temperatures.

The indefinite article "a" or "an" and its corresponding definite article "the" as used herein means at least one, or one or more, unless specified otherwise.

Abbreviations, which are well known to one of ordinary skill in the art, may be used (e.g., "h" or "hr" for hour or hours, "g" or "gm" for gram(s), "mL" for milliliters, and "rt" for room temperature, "nm" for nanometers, and like abbreviations).

Specific and preferred values disclosed for components, ingredients, additives, reactants, reagents, polymers, oligomers, monomers, times, temperatures, and like aspects, and ranges thereof, are for illustration only; they do not exclude other defined values or other values within defined ranges. The compositions and methods of the disclosure include those having any value or any combination of the values, specific values, more specific values, and preferred values described herein.

In embodiments of the disclosure, the problem of immobilizing, isolating, detecting, characterizing interactions, or other like dispositions of target proteins, and like substances, can be overcome by, for example, chemically modifying the surface properties of the target protein or protein mixture of interest and the immobilizing surface. A maleimide-linker-modified protein can then be readily and selectively immobilized, isolated, detected, or other like dispositions, on a thiol-modified polymer coated surface, as defined herein.

During our researches on biosensor surface chemistries, it was discovered that a target protein could be chemically modified, by for example, converting one or more amine groups to, for example, an organic amide group having a maleimide substituent spatially separated by a linker unit. The chemically modified protein could then be readily immobilized on a thiol-modified polymer coated substrate surface for detection, analysis, or like processing or characterization.

In embodiments, the disclosure provides compositions, articles, and methods for making and using a target protein.

In embodiments, the disclosure provides a biosensor surface having thiol functionality that can be, for example, derived or prepared from a commercially available derivatized ethyl-maleic anhydride (dEMA) copolymer surface, such as an Epic® multi-well plate available from Corning, Inc. In embodiments, the thiol functionality on the biosensor surface can be prepared, for example, in two steps.

In embodiments, the disclosure provides an immobilization method for attaching target ligands, such as proteins, and like biological or chemical entities of interest, to a thiol-functionalized biosensor surface.

In embodiments, a maleimide functionalized protein can be immobilized onto a thiol-functionalized biosensor surface. The protein is immobilized according to the disclosed process in an amount sufficient to permit an activity assay with the immobilized proteins.

Current Epic® biochemical assays can use, for example, amine and maleic anhydride or amine and activated acid coupling reactions to immobilize proteins onto the surfaces of multi-well plates. These reactions can be simple and widely applicable to a large number of proteins. Disadvantages of the reactions can include, for example, non-specificity, competition with hydrolysis, and the number of bonds between the surface and the protein cannot be easily controlled. These disadvantages can create difficulties in controlling the amount of protein immobilized and can lead to proteins that are too tightly bound to the surface, which effects can cause reduced activity or denaturing. To address these issues and to further expand the scope of Epic® instrument application, different chemistries that can link proteins and biomolecules to a modified surface of the multi-well plates were considered. For several of these alternative chemistries a thiol-modified surface can be used. Immobilization to a thiol-modified surface can be accomplished using a specific coupling reaction. Such coupling reactions include, for example, a thiol-maleimide coupling reaction. The SH-maleimide

coupling reaction has been used in the field of bioconjugation (for a review see Comb. Chem. & High Throughput Screening, 2004, 7, 213-221). The SH-maleimide coupling can readily occur at room temperature in a biological environment. Although not bound by theory, the SH-maleimide coupling reaction is believed to be highly specific because the maleimide functionality is not found on biomolecules, and cysteine residues are rarely found exposed on the outside of a protein.

In SPR, preparing a thiol surface through reaction of a thiol containing reagent such as cystamine to a surface bound polymer, typically a carboxymethyldextran, is known *(*Biosensors & Bioelectronics, 1995, 10, 813-8122).

In embodiments, the disclosed method for making a thiol-modified surface can be applied to a commercially available, pre-activated dEMA surface, and adapted for particulars of the Epic® multiwell-plates.

In embodiments, advantages of present disclosure, that is a thiol-modified surface and immobilization method compared to, for example, a maleic anhydride or activated carboxylic acid surface include, for example:

the surface is specific as illustrated by certain unmodified proteins that have no or very low binding to the thiol surface;
the number of bonds formed between the thiol surface matrix and the target protein can be controlled by the stoichiometry of a linker unit attached to the protein prior to immobilization (certain linker:protein ratios can provide superior immobilization and subsequent binding results);
hydrolysis does not limit protein immobilization;
the disclosed process is applicable to surfaces; and
the disclosed surface and process expand the available materials and methods available for use in optical resonant waveguide biosensing.

In embodiments, the disclosure provides a method to immobilize a protein, including, for example:

combining the protein and a mixture comprised of an activated spacer having a maleimide group in a buffer solution, to form a maleimide-modified protein; and
contacting the maleimide-linker-modified protein and a buffer swollen, thiol-modified, surface bound, polymer to immobilize the maleimide-linker -modified protein on the polymer surface.
The method can further comprise contacting the surface immobilized protein with a ligand, and further optionally detecting ligand binding by the surface immobilized protein. The detected ligand binding by the surface immobilized protein can be, for example, from about 0.1 to about 5,000 picometers (pm), and the detected ligand binding by the surface immobilized protein can be, for example, from about 1 to about 1,000 picometers (pm), including intermediate values and ranges. The protein can be, for example, immobilized at from about 300 to about 5,000 picometers (pm), including intermediate values and ranges. The protein and the activated spacer can be, for example, in a relative mole ratio of from about 1:1 to about 50:1, including intermediate values and ranges.

In embodiments, the activated linker can be, for example, at least one compound of the formula:

**B-$R^1$-C**

where
**B** comprises a monovalent Micheal acceptor group, such as an alpha, beta-unsaturated carbonyl; for example, a maleimide, which reacts second with the thiol-modified surface;
**C** comprises a monovalent reactive group, such as a succinimidyl ester, which reacts first with an amine of the protein; and
**-$R^1$-** can be, for example, a divalent spacer group selected from a branched or unbranched, substituted or unsubstituted $(C_{1-20})$hydrocarbylene, or an oxyhydrocarbylene glycol of the formula $-CHR^3-CHR^3-(O-CHR^3-CHR^3)_z-$, or a combination thereof, $R^3$ is H, or a branched or unbranched, substituted or unsubstituted $(C_{1-8})$alkyl, and z is an integer of from 1 to about 10.
The **B** can be, for example, a maleimide group, C can be, for example, a succinimidyl ester group, and **-$R^1$-** can be, for example, a $(C_{1-20})$hydrocarbylene. The disclosed protein immobilization method can have, for example, a Z' value of from about 0.3 to about 1.0 for the protein immobilized on the surface, and a Z' value of from about 0.3 to about 0.6 for the protein immobilized on the surface.
In embodiments, the **B** can be a maleimide group, **C** is a succinimidyl ester group, and **-$R^1$-** is a branched or unbranched, substituted or unsubstituted $(C_{1-20})$hydrocarbylene.

In embodiments, the immobilization method can further comprise, for example, rinsing the protein contacted substrate with buffer to remove free protein, rinsing the ligand contacted substrate with buffer to remove free ligand, or a combination thereof. The detecting can be accomplished, for example, with a resonant waveguide grating biosensor, or like biosensor detectors.

In embodiments, the disclosure provides a thiol-modified surface article prepared by the process comprising:

contacting a carboxy polymer surface with a protected-thiol source reagent to form a protected disulfide group, and deprotecting the protected disulfide group.

In embodiments, the disclosure provides an article comprising the thiol-modified surface as described above. In embodiments, the article can further comprise at least one protein immobilized on the surface. In embodiments, the article can further comprise at least one ligand bound to the immobilized protein on the surface.

In embodiments, the disclosure provides surface attached polymers or copolymers that include the general formula **(I):**

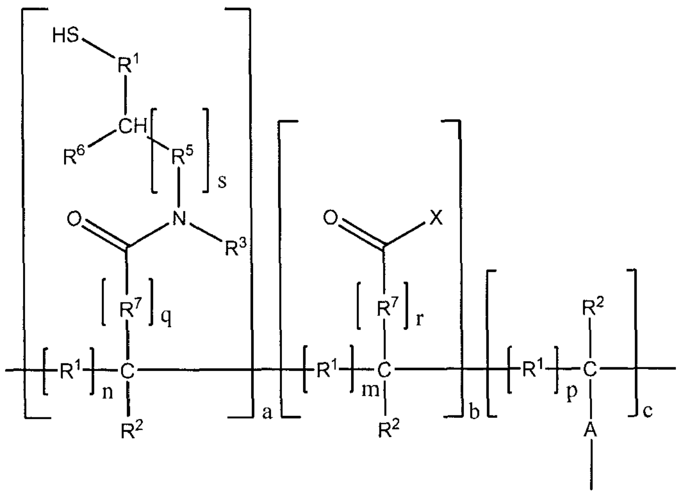

**(I)**

where

**A** is a tie-layer attachment group;

***a*** comprises a thiol-containing mer, the thiol being suitable to immobilize a maleimide-modified bioentity;
***b*** comprises an amide- or a carboxylic acid-containing mer;
***c*** comprises at least one mer having the tie-layer attachment group **A;**
***a,*** *b,* and c are each independently from 1 to about 100,000;
***m***, ***n***, ***p***, ***q***, ***r***, and ***s*** are each independently 0 or 1 (in all instances, when ***m***, ***n***, ***p***, ***q***, ***r***, or ***s*** are 0, a single covalent bond is recognized to connect groups adjacent to the intervening R substituent(s));
**X** is -N($R^3$)($R^4$), or -O$R^3$;
$\mathbf{R^1}$ is a divalent ($C_{1-4}$)hydrocarbyl;
$\mathbf{R^2}$ is H or monovalent ($C_{1-4}$)hydrocarbyl;
$\mathbf{R^3}$ and $\mathbf{R^4}$ are each independently -H, or a monovalent substituted or unsubstituted ($C_{1-10}$)hydrocarbyl;
$\mathbf{R^5}$ is a divalent alkyl amide or alkoxylated amide spacer of the formula - $(CH_2)_u$-C(O)N($R^3$)- or -$(CH_2CH_2O)_u$-C(O) N($R^3$)-, where **u** is an integer from 1 to 10;
$\mathbf{R^6}$ is independently -H or -COOH; and
$\mathbf{R^7}$ is a carbon-carbon bond or a divalent substituted or unsubstituted ($C_{1-4}$) hydrocarbyl,
and a salt thereof, or a combination of a non-salt and a salt.

A specific example of a thiol containing polymer that can be prepared when, for example, a copoly (alkyl-maleic anhydride) such as copoly (ethyl-maleic anhydride) (EMA) is selected as the starting polymer, is of the formula **(II):**

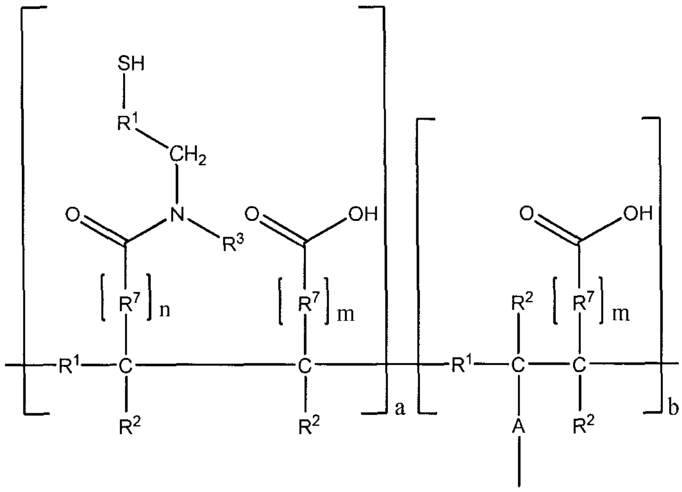

**(II)**

where

***a*** comprises a half-thiol half-acid containing mer, the thiol being suitable to immobilize a maleimide-modified bioentity;
***b*** comprises at least one mer having a tie-layer attachment group **A;**
***a*** and ***b*** are each independently integers from 1 to about 100,000;
***n*** and ***m*** are each independently either 0 or 1;
$\mathbf{R^1}$ is a divalent substituted or unsubstituted $(C_{1-10})$hydrocarbyl;
$\mathbf{R^2}$ is a H or $(C_{1-4})$hydrocarbyl;
$\mathbf{R^3}$ is -H or a monovalent substituted or unsubstituted $(C_{1-10})$hydrocarbyl, for example, $-CH_2CH_2OH$, $-CH_2CH_2OCH_3$, PEG, and like substituents; and
$\mathbf{R^7}$ is a carbon-carbon bond or substituted or unsubstituted $(C_{1-4})$ hydrocarbyl, and a salt thereof, or a combination of a non-salt and a salt.

A specific example of a thiol containing polymer that can be prepared when, for example, a derivatized copoly (alkyl-maleic anhydride) such as copoly (ethyl-maleic anhydride) (dEMA) is selected as the starting polymer, is of the formula **(III):**

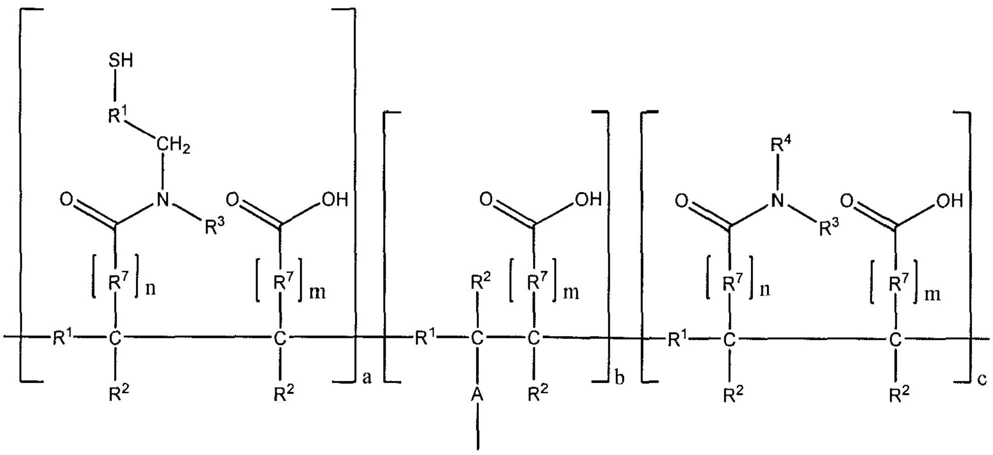

**(III)**

where

***a*** comprises a half-thiol half-acid containing mer, the thiol being suitable to immobilize a maleimide-modified bioentity;
***b*** comprises at least one mer having a tie-layer attachment group **A;**
***c*** comprises a half-amide half-acid containing mer;

***a***, *b,* and c are each independently integers from 1 to about 100,000;
***n*** and ***m*** are each independently 0 or 1;
**$R^1$** is a divalent substituted or unsubstituted $(C_{1-10})$hydrocarbyl;
**$R^2$** is a H or $(C_{1-4})$hydrocarbyl;
**$R^3$** and **$R^4$** are each independently -H or a monovalent substituted or unsubstituted $(C_{1-10})$hydrocarbyl, for example, $-(CH_2)_6-OCH_3$, $-CH_2CH_2OH$, $-CH_2CH_2OCH_3$, polyethylene glycol (-PEG), and like substituents; and
**$R^7$** is a carbon-carbon bond or substituted or unsubstituted $(C_{1-4})$ hydrocarbyl, and a salt thereof, or a combination of a non-salt and a salt.

A specific example of a polymer of the formula **(III)** is a surface attached polymer comprised of mers of the formula **(IIIa):**

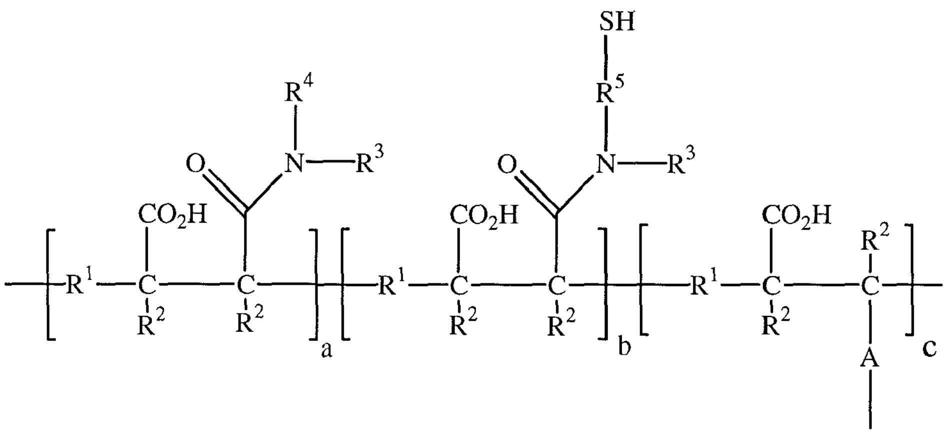

**(IIIa)**

where:

***a*** comprises a half-acid half-amide mer;
***b*** comprises a half-acid half-thiol mer, the half-thiol being suitable to immobilize a maleimide-modified bioentity;
***c*** comprises at least one mer having a tie-layer attachment group **A;**
***a***, *b,* and c are each independently an integer from 1 to 100,000;
**$R^1$** is $(C_{1-4})$hydrocarbyl;
**$R^2$** is H or $(C_{1-4})$hydrocarbyl;
**$R^3$** and **$R^4$** are each independently a monovalent -H or $-(C_{1-10})$hydrocarbyl; and
**$R^5$** is a divalent $(C_{1-10})$hydrocarbyl, and a salt thereof, or a combination of a non-salt and a salt.

A specific example of a thiol containing polymer that can be prepared when, for example, a PEG functionalized dEMA-type polymer ("dEMA-spacer-thiol" modified polymer) is selected as the starting polymer, and when attached or otherwise associated with the surface, is of the formula **(IV):**

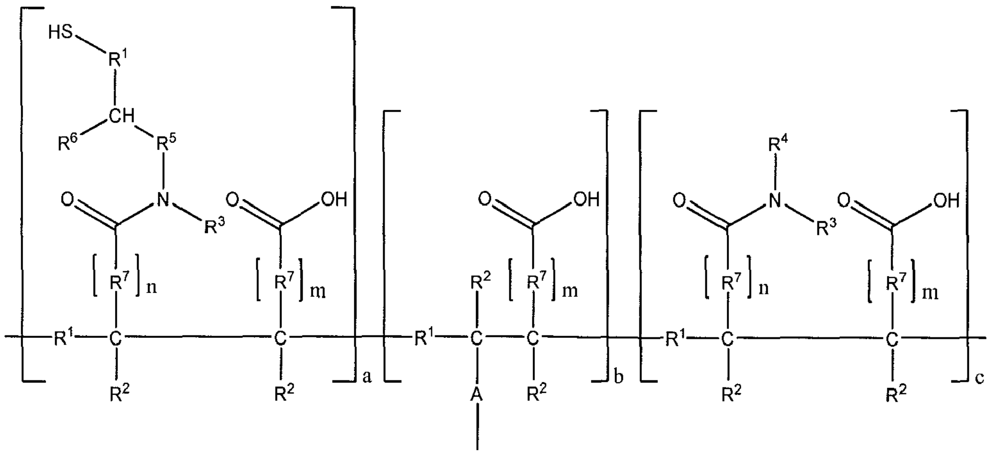

**(IV)**

where

***a*** comprises a half-thiol half-acid containing mer;
***b*** comprises at least one mer having a tie-layer attachment group **A**;
***c*** comprises a half-amide half-acid containing mer;
***a, b,*** and ***c*** are each independently an integer from 1 to about 100,000;
***n*** and ***m*** are each independently 0 or 1;
**$R^1$** is a divalent substituted or unsubstituted $(C_{1-10})$hydrocarbyl;
**$R^2$** is a H or monovalent $(C_{1-4})$hydrocarbyl;
**$R^3$** and **$R^4$** are each independently -H or a monovalent substituted or unsubstituted $(C_{1-10})$hydrocarbyl;
**$R^5$** is a carbon-carbon bond or a divalent substituted or unsubstituted$(C_{1-10})$ hydrocarbyl, $-(CH_2)_t-$, $(CH_2CH_2O)_{t-}$,$-(CH_2)_t-C(O)N(R^3)-$, or $-(CH_2CH_2O)_t-C(O)N(R^3)-$ where t is an integer of 1 to 10;
**$R^6$** is -H or -COOH; and
**$R^7$** is a carbon-carbon bond or divalent substituted or unsubstituted$(C_{1-4})$ hydrocarbyl, and a salt thereof, or a combination of a non-salt and a salt.

A specific example of the thiol containing polymer of formula **(IV)** is of the formula **(IVa):**

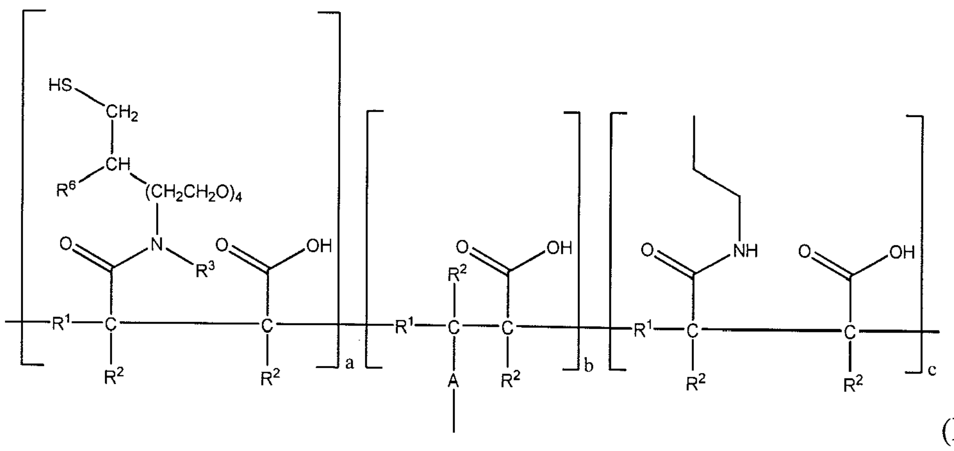

**(IVa)**

A specific example of a thiol containing polymer that can be prepared when, for example, polyacrylic acid (PAA) is selected as the starting polymer, and attached to the surface is of the formula **(V):**

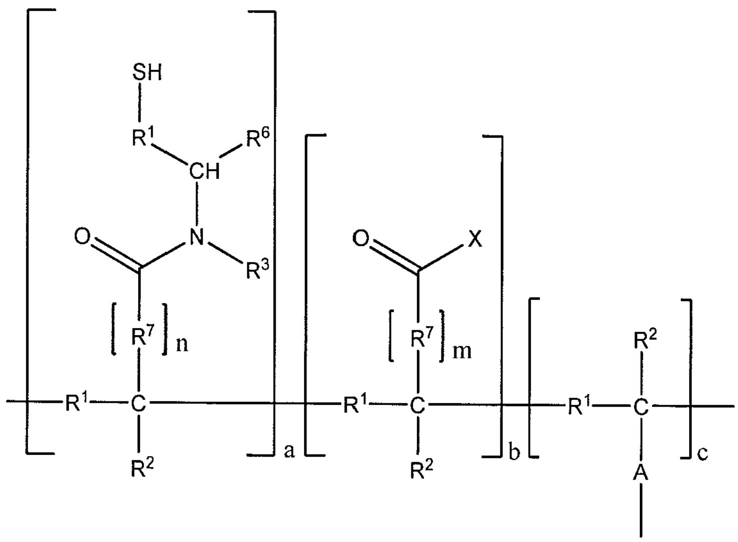

(V)

where

***a*** comprises a thiol-containing mer;
***b*** comprises an acid-containing mer;
***c*** comprises at least one mer having a tie-layer attachment group A;
***a, b,*** and ***c*** are each independently an integer from 1 to about 100,000;
***n*** and ***m*** are each independently 0 or 1;
**X** is $-N(R_3)(R_4)$, or $-OR_3$;
$\mathbf{R^1}$ is $(C_{1-4})$hydrocarbyl;
$\mathbf{R^2}$ is H or $(C_{1-4})$hydrocarbyl;
$\mathbf{R^3}$ is -H or a monovalent substituted or unsubstituted $(C_{1-10})$hydrocarbyl;
$\mathbf{R^6}$ is -H or an -COOH group; and
$\mathbf{R^7}$ is a carbon-carbon bond or substituted or unsubstituted$(C_{1-4})$ hydrocarbyl, and a salt thereof, or a combination of a non-salt and a salt.

A specific example of a thiol containing polymer that can be prepared when, for example, a copoly(acrylic acid-methacrylamide) **(AA/MAm)** is selected as the starting polymer, is of the formula **(VI):**

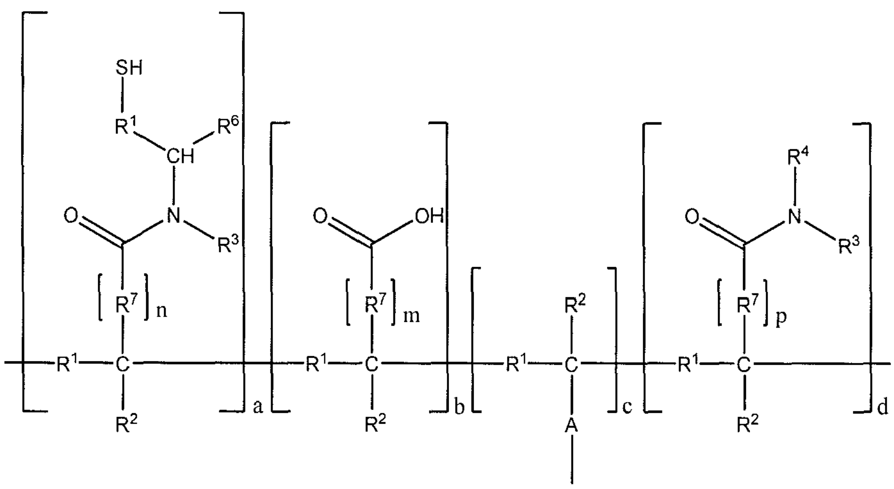

(VI)

where

***a*** comprises a thiol containing mer;
***b*** comprises an acid containing mer;
***c*** comprises at least one mer having a tie-layer attachment group **A;**
***d*** comprises an amide containing mer;
***a, b,*** and ***c*** are each independently an integer from 1 to about 100,000;
***m***, ***n***, and ***p*** are each independently 0 or 1;
$\mathbf{R^1}$ is $(C_{1-4})$hydrocarbyl;
$\mathbf{R^2}$ is H or $(C_{1-4})$hydrocarbyl;
$\mathbf{R^3}$ and $\mathbf{R^4}$ are each independently **-H** or a monovalent substituted or unsubstituted $(C_{1-10})$hydrocarbyl;
$\mathbf{R^6}$ is -H or an -COOH group; and
$\mathbf{R^7}$ is a carbon-carbon bond or substituted or unsubstituted$(C_{1-4})$ hydrocarbyl, and a salt thereof, or a combination of a non-salt and a salt.

A specific example of Formula **(VI)** is a polymer of the formula **(VIa):**

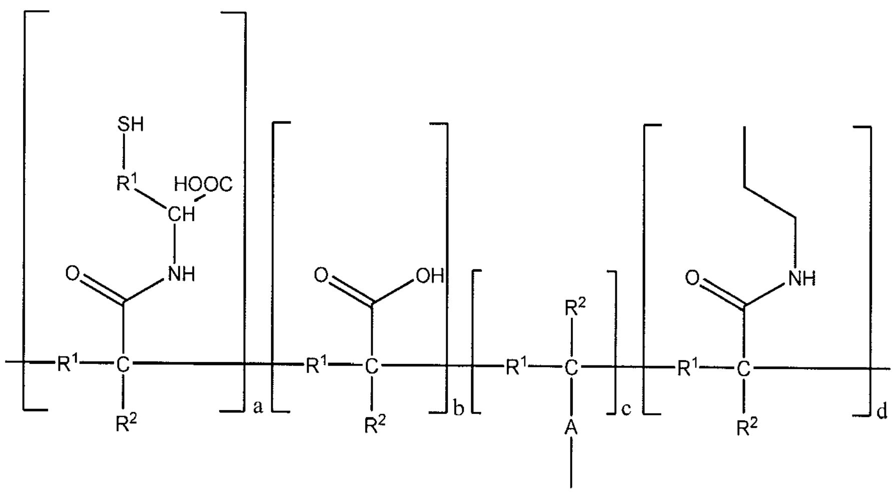

**(VIa)**

where $\mathbf{R^1}$, $\mathbf{R^2}$, ***a, b, c, d,*** and **A** are as defined above.

**Table 1. Summary of polymers prepared and tested.**

| Formula | $R^1$ | $R^2$ | $R^3$ & $R^4$ | $R^5$ | $R^6$ | $R^7$ |
|---|---|---|---|---|---|---|
| **(III)** | $(CH_2)_2$ | H | H & propyl | - | - | ***n*** = 0 |
| **(IV)** | $(CH_2)_2$ | H | H & propyl | $(PEG)_4$ spacer | both H and COOH | ***n, m*** = 0 |
| **(VI)** | $(CH_2)_2$ | H | H & H | | both and H COOH | ***n, m, p*** = 0 |

The carboxy polymer surface can be, for example, a reaction product of a poly(alkylene-alt-maleic anhydride) copolymer and an alkyl-amine. More specifically, the carboxy polymer surface can be, for example, a poly(ethylene-alt-maleic anhydride) having from about 30 to about 50 mol% of the anhydride groups reacted with propylamine, or a like alkylamine or a substituted alkylamine. In embodiments, the thiol-modified surface can be, for example, a modified carboxy polymer surface comprising a poly(ethylene-alt-maleic anhydride) having from about 60 to about 100 mol% of the anhydride groups reacted with an alkylamine, a thioalkylamine, or a combination thereof. In embodiments, the thiol-modified surface can be prepared in a two step sequence where, for example, in a first step an initial carboxy polymer surface comprising a poly(ethylene-alt-maleic anhydride) can have from about 30 to about 50 mol% of the anhydride groups reacted with an alkylamine, and in a second step the alkylamine modified poly(ethylene-alt-maleic anhydride) can have from about 50 to about 70 mol% of any residual or remaining anhydride groups that were not consumed in the reaction with the alkylamine are reacted with a thioalkylamine. Thus, in embodiments, the thiol-modified surface can comprise a carboxy polymer surface comprising a poly(ethylene-alt-maleic anhydride) having from about 30 to about

50 mol% of the initial anhydride groups reacted with an alkylamine, and from about 50 to about 70 mol% of the remaining or residual anhydride groups reacted with a thioalkylamine.

The carboxy polymer surface can be, for example, a poly(acrylic acid) or a copolymer of an acrylic acid monomer and a second monomer, such as copoly(acrylic acid-acrylamide), and a blocking agent. The blocking agents, can be, for example, 2-(2-aminoethoxy) ethanol, *N*,*N*-dimethyl ethylenediamine, ethanolamine, ethylenediamine, hydroxyl amine, methoxyethyl amine, ethyl amine, isopropyl amine, butyl amine, propyl amine, hexyl amine, 2-amino-2-methyl-1-propanol, 2-(2-aminoethyl amino) ethanol, 2-(2-aminoethoxy)ethanol, dimethylethanolamine, dibutyl ethanolamine, 1-amino-2-propanol, polyethylene glycol, polypropylene glycol, 4,7,10-trioxa-1,13-tridecanediamine, polyethylene glycol, or an amine-terminated-polyethylene glycol, Trizma® hydrochloride, or any combination thereof. Many of the above mentioned blocking agents can be used as a spacer as defined herein.

In embodiments, the linker and the protein can be, for example, in a relative mole ratio of from about 1:1 to about 50:1, including intermediate values and ranges.

In embodiments, a suitable buffer can be selected, for example, based upon the pH at which one desires to operate. Typical buffers can include, for example, 20 mM sodium citrate for a pH of about 2.5 to about 5.6, 20 mM sodium acetate for a pH of about 3.7 to about 5.6, sodium phosphate for a pH of about 6.0 to about 9.0, and like buffer and pH ranges, or combinations thereof, including intermediate values and ranges. Other suitable buffers can include, for example, tris(hydroxymethyl)aminomethane (tris) for pH of about 7.0 to about 9.2, and (4-(2 hydroxyethyl)piperazine-1-ethanesulfonic acid (HEPES) for a pH of about 6.8 to about 8.2. Still other buffers are disclosed in, for example, the CRC Handbook, section 8-42.

In embodiments, the protein can be present, for example, in an amount of from about 0.001 micrograms/mL to about 1,000 micrograms/mL, from about 1 micrograms/mL to about 100 micrograms/mL, and from about 10 micrograms/mL to about 100 micrograms/mL, and like amounts, including intermediate values and ranges. The maleimide-linker-modified protein can be immobilized on the thiol-modified polymer surface, for example, in an amount having an indication of from about 100 to about 10,000 picometers, from about 200 to about 5,000 picometers, and from about 400 to about 5,000 picometers, including intermediate values and ranges, as measured by, for example, a resonance wave guide biosensor, such as a Corning, Inc., Epic® instrument, and like biosensor instruments or systems.

Many major classes of proteins, for example, kinases, proteases, nuclear receptors, and like proteins, can be immobilized according to the methods of present disclosure.

In embodiments, the method can further include forming at least one covalent bond between the substrate surface and the modified protein, for example, to provide covalent immobilization of the derivatized protein.

In embodiments, the method can further include rinsing the maleimide-linker-modified protein contacted substrate with buffer to remove, for example, unbound or free protein, such as modified or derivatized protein which is only weakly associated with the polymer coated surface and not immobilized on the polymer coated substrate.

In embodiments, the disclosure provides a method for detecting a protein including, for example:

modifying the protein by contacting the protein with a mixture of an activating agent and an maleimide-spacer-amine compound to form a maleimide-spacer modified protein;
contacting a thiol-modified polymer surface and the maleimide-linker-modified protein to immobilize the maleimide-linker-modified protein on the thiol-modified surface; and
detecting the immobilized maleimide-spacer modified protein.

In embodiments, the detection of the immobilized the maleimide-linker-modified protein on the thiol-modified surface can be accomplished, for example, with a resonant waveguide grating biosensor and like biosensors.

In embodiments, the thiol-modified polymer coated surface can be, for example, a reaction product of a poly (alkylene-maleic anhydride), such as a poly(alkylene-alt-maleic anhydride), such as being previously associated with the surface of a biosensor with, for example, and aminosilane or like conversion agents, and an alkyl-amine, on the surface of a microwell plate biosensor. Such amine-modified polymer coated surfaces having a poly(alkylene-alt-maleic anhydride) or an amine derivative are commercially available from, for example, Corning, Inc, such as in the Epic® microplate.

The thiol-modified surface can comprise a carboxy polymer surface comprising, for example, a poly(ethylene-alt-maleic anhydride) having from about 30 to about 50 mol% of the anhydride groups reacted with propylamine. The protein can be present in an amount of, for example, from about 10 micrograms/mL to about 100 micrograms/mL, and the maleimide-linker-modified protein can be immobilized on the substrate, for example, at an indication of from about 1 to about 5,000 picometers, and from about 1 to about 1,000 picometers, including intermediate values and ranges, as measured by a resonance wave guide biosensor.

In embodiments, the carboxy polymer coated surface can be, for example, at least one of:

an alkyl-amine modified poly(alkylene-alt-maleic anhydride);

a poly(acrylic acid); or
a copoly(acrylic acid-acrylamide); the polymer having a plurality of **-S-H** reactive groups capable of reacting with a maleimide-modified target, and a plurality of ionizable groups, and at least one of the **-S-H** reactive groups has a spacer unit situated between the polymer backbone and the **-S-H** reactive group; and like polymers, or a combination of two or more of the polymers, and a salt thereof, or a combination of a non-salt and a salt.

In embodiments, the carboxy polymer can comprise, for example, mers of the formula:

**- {- CH (C (=O) (OH))-CH (C (=O) NH- R2-(X))-R1-}m-**

where
**$R^1$** is a divalent -($C_{2-6}$ alkylene)-;
**$R^2$** is a divalent spacer comprising from about 6 to about 30 atoms;
**X** is a **-S- H** reactive group or a salt thereof; and
**m** is from about 10 to about 10,000,
or a salt thereof.

A carboxy polymer having the above mentioned **$R^2$** comprising a divalent spacer comprising from about 6 to about 30 atoms, is disclosed, for example, in commonly owned and assigned copending application U.S. Serial No. 61/123609, filed April 10, 2008.

In embodiments, the carboxy polymer can be, for example, a copolymer comprised of mers of the formula:

**- {- CH (C (=O) (OH))-CH (C (=O) NH- R2-(X))-R1-}m-**

**- {- CH (C (=O) (OH))-CH (C (=O)-(Y))-CH- R1-}n-**

**- {- CH (C (=O) (OH))-CH (C (=O) NH- R3)-R1-}o-**

where
**$R^1$** is a divalent -($C_{2-6}$ alkylene)-;
**$R^2$** is a divalent spacer comprising from about 6 to about 30 atoms;
**$R^3$** is a monovalent -($C_{1-6}$ alkyl);
**X** is a **-S- H** reactive group or a salt thereof;
**Y** is a surface substantive group or a salt thereof;
***m*** is from about 10 to about 10,000;
***n*** is from about 10 to about 10,000; and
***o*** is from about 10 to about 10,000,
or a salt thereof.

In embodiments, the mers and substituents of a specific spacer containing polymer can be, for example:

**$R^1$** is a -(-$CH_2$-$CH_2$-)-;
$R^2$ is a divalent spacer comprising a poly alkylene glycol segment containing from about 2 to about 6 alkylene glycol units, such as -$CH_2$-$CH_2$-(O-$CH_2$-$CH_2$-)$_{2-6}$-;
**$R^3$** is a propyl group;
**X** is a sulfo-N-hydroxysuccinimide group or the salt thereof;
**Y** is a surface substantive group;
***m*** is from about 10 to about 10,000;
***n*** is from about 10 to about 10,000; and
***o*** is from about 10 to about 10,000,
and a salt thereof the polymer having a plurality of **-S-H** reactive groups capable of reacting with a maleimide-modified target, and a plurality of ionizable groups, and at least one of the **-S-H** reactive groups has a spacer unit situated between the polymer backbone and the **-S-H** reactive group; and like polymers, or a combination of two or more of the polymers, and a salt thereof, or a combination of a non-salt and a salt.

In embodiments, a more specific spacer can have mers of, for example, an **$R^2$** having a divalent spacer of the formula:

**-NR3-(CH2)2-(O- CH2-CH2)p-C (=O)-O-**

where each $R^3$ is hydrogen or a monovalent -($C_{1-6}$ alkyl), and ***p*** is from about 2 to about 6.

In embodiments, the maleimide-linker-NHS reagent and the protein can be in a relative mole ratio of, for example, from about 1:1 to about 50:1, from about 2:1 to about 25:1, and from about 5:1 1 to about 10:1, including intermediate values and ranges.

In embodiments, the protein for immobilization and detection can be present in an amount of, for example, from about 10 micrograms/mL to about 100 micrograms/mL, and the resulting maleimide-linker-modified protein can be immobilized on the substrate in an amount, for example, having an indication from about 100 to about 10,000 picometers (pm or dimensionless relative units), from about 200 to about 7,500 pm, from about 400 to about 5,000 pm, from about 500 to about 2,500 pm, and from about 500 to about 1,000 picometers (pm), including intermediate values and ranges, as measured by, for example, a resonant waveguide Epic® instrument, including intermediate values and ranges.

In embodiments, the immobilized protein can be associated with the thio-modified carboxy polymer coated surface by, for example, covalent, ionic, hydrophobic, or a combination of these and other associations.

In embodiments, the disclosure also provides an article having an immobilized protein thereon, and to methods of using the articles having an immobilized protein on the article's surface, such as in biosensing application, and like applications.

In embodiments, the disclosure provides supports useful for performing assays. In embodiments, a support for performing an assay comprises a substrate having a polymer directly or indirectly attached to the substrate, the polymer having a plurality of **-S- H** reactive groups that are capable of attaching-to or binding-with a maleimide-modified biomolecule and a plurality of ionizable groups capable of attracting the maleimide-modified biomolecule to the **-S- H** modified surface of the substrate, the ratio of **-S- H** reactive groups to ionizable groups can be, for example, from about 0.5 to about 10, and the polymer can have a spacer unit or spacer groups situated between the polymer backbone and all or at least some of the **-S- H** reactive groups. In instances where the support is used, for example, for label independent detection methods, the polymer need not contain a photoactive group.

In embodiments, the disclosure provides an article for label-independent detection, the article comprising:

a substrate comprising a biomolecule-reactive contact surface comprising a plurality of **-S-H** groups; and
optionally a tie layer having the abovementioned biomolecule-reactive contact surface composition deposited on the tie layer.

The substrate can further include one or more maleimide-modified biomolecules attached to any of the biomolecule immobilization groups, i.e., the -S-H reactive groups.

In embodiments, the disclosure provides an article for label independent detection, the article comprising:

a substrate having a polymer coated contact surface comprising:

a tie layer; and
a thiol-modified polymer coat having a plurality of **-S-H** reactive groups; and

optionally a maleimide-modified biomolecule attached to the contact surface through one or more of the **-S-H** reactive groups.

In embodiments the disclosure provides an apparatus for label independent detection, the apparatus comprising: an optical biosensor having the aforementioned article having a thiol-modified polymer coated contact surface.

In embodiments, the disclosure provides an apparatus for label-independent detection, the apparatus comprising: an optical biosensor having a biomolecule-reactive thiol-modified contact surface comprising the abovementioned contact surface and optionally one or more maleimide-modified biomolecules attached to or reacted with any of the **-S-H** reactive groups, i.e., biomolecule immobilization groups.

Suitable substrates can include, for example, a microplate, a slide, or any other material that is capable of attaching to the polymer. In embodiments, when the substrate is a microplate, the number of wells and well volume may vary depending upon, for example, the scale and scope of the analysis. Other examples of useful substrates can include, for example, a cell culture surface such as a 384-well microplate, a 96-well microplate, 24-well dish, 8-well dish, 10 cm dish, a T75 flask, or like articles.

For optical or electrical detection applications, the substrate can be, for example, transparent, impermeable, or reflecting, and electrically conducting, semiconducting, or insulating. For biological applications, the substrate material can be, for example, either porous or nonporous, and can be selected, for example, from organic or inorganic materials, or a combination thereof.

In embodiments, the substrate can be, for example, a plastic, a polymeric or copolymeric substance, a ceramic, a glass, a metal, a crystalline material, a noble or semi-noble metal, a metallic or non-metallic oxide, an inorganic oxide,

an inorganic nitride, a transition metal, and like materials, or any combination thereof. Additionally, the substrate can be configured so that it can be placed in any detection device. In one aspect, sensors can be integrated into, for example, the bottom or underside of the substrate and used for subsequent detection. These sensors can include, for example, optical gratings, prisms, electrodes, quartz crystal microbalances, and like articles. Detection methods can include, for example, fluorescence, phosphorescence, chemiluminescence, refractive index, mass, electrochemical, and like detection methods. In embodiments, the substrate can be a resonant waveguide grating sensor.

In embodiments, the substrate can include an inorganic material. Examples of inorganic substrate materials can include, for example, metals, semiconductor materials, glass, ceramic materials, and like materials. Examples of metals that can be used as substrate materials include, for example, gold, platinum, nickel, palladium, aluminum, chromium, steel, gallium arsenide, or combination thereof. Semiconductor materials used for the substrate material can include, for example, silicon and germanium. Glass and ceramic materials used for the substrate material can include, for example, quartz, glass, porcelain, alkaline earth aluminoborosilicate glass and other mixed oxides. Further examples of inorganic substrate materials can include, for example, graphite, zinc selenide, mica, silica, lithium niobate, and inorganic single crystal materials. In embodiments, the substrate can be gold or gold coated, for example, a gold sensor chip.

In embodiments, the relative mole ratio of thiol reactive groups such as **-S-H,** also referred to as a thiol group, a sulfhydryl group, a mercapto- or mercaptan group, which reactive groups are capable of reacting with a maleimide-modified biomolecule, to ionizable groups, such as carboxyl -C(=O)(OH) groups, can be, for example, from about 0.01 to about 100 ($0.01 \leq R/I \leq 100$, where R/I represents the ratio of reactive to ionizable groups), and from about 0.1 to about 10. Reactive groups can be distinguished from ionizable (i.e., ionic or ionized) groups, for example, based at least on a greater relative reactivity (reaction rate) a reactive group compared to an ionizable group, such as having a relative reaction rate of from about 2 to about 10,000 times, from about 5 to about 1,000 times, and from about 10 to about 100 times greater than the ionizable group.

When the number of thiol reactive groups in the polymer is, for example, less than about 1% of all possible reactive and ionizable sites, the attachment of the biomolecules, while still possible will be less than highly efficient. Additionally or alternatively, if there is less than about 1% ionizable groups available of all possible reactive and ionizable sites, the attachment of the biomolecules and subsequent ligand binding to the bound biomolecules will be less than highly efficient compared to when higher amounts of thiol reactive and ionizable groups are present.

In embodiments, the relative mole ratio of mers (***a*:*b*:*c***) of the disclosed structural formulas can be, for example, where c contains the attachment group A, then ***a* > *b* >>** $a > b >> c$, $a \cong b >> c$, $b > a >> c$, **>> *c*, > *a* ,** and $c \neq 0$. In embodiments, the relative mole ratio of mers (***a***:***b***) of the disclosed structural formulas can be, for example, $a > b$, $b > a$, $a \cong b$, $a >> b$, or $b >> a$. In embodiments, when ***b*** contains the attachment group A, then for example, $a > c << b$, $a \cong c >> b$, $c > a >> b$, and $b \neq 0$. In embodiments, the relative mole ratio of thiol reactive groups to ionizable groups (R/I) of the polymer attached to the surface can be, for example, from about 0.5 to about 5.0. In embodiments, the lower end of the ratio can be, for example, 0.5, 0.6, 0.7, 0.8, 0.9, 1.0, 1.5, 2.0, 2.5, 3.0, 3.5, 4.0, 4.5, or 5.0 and the upper end can be, for example, 2.0, 2.5, 3.0, 3.5, 4.0, 4.5, 5.0, 5.5, 6.0, 6.5, 7.0, 7.5, 8.0, 8.5, 9.0, 9.5, or 10.0, where any lower and upper end can form the ratio range. In embodiments, the ratio of thiol reactive groups to ionizable groups can be, for example, from about 0.5 to 9.0, about 0.5 to 8.0, about 0.5 to 7.0, about 0.5 to 6.0, about 0.5 to 5.0, about 0.5 to 4.0, about 0.5 to 3.0, about 0.6 to 3.0, about 0.65 to 3.0, or about 0.67 to about 3.0, including intermediate values and ranges.

The formation and number of thiol reactive groups and ionizable groups present on the surface polymer can be controlled in a number of ways. In embodiments, the polymer can be synthesized from monomers possessing thiol reactive groups and monomers having ionizable groups. Alternatively, non-thiol reactive groups, for example, anhydride groups, such as polymerized or copolymerized maleic anhydride moieties or like anhydride moieties, can be derivatized in solution or subsequent to attachment of the polymer to the surface to form the thiol reactive groups. In embodiments, the stoichiometry of the monomers selected can control the ratio of thiol reactive groups to ionizable groups. In embodiments, a polymer possessing just thiol reactive groups can be treated so that some of the reactive groups are converted to ionizable groups prior to attaching the polymer to the substrate, for example, blocking the thiol by an alkylation reaction with lesser molar equivalents of a halogenated carboxylic acid salt, such as $X'-CH_2-CH_2-CO_2M$, where X' is halide and M is a metal cation to form $-S-CH_2-CH_2-CO_2M$ groups. The starting polymer can be commercially available or synthesized using known techniques, and modified in accordance with the present disclosure. In embodiments, a polymer can be attached to the substrate, and the attached polymer can be treated with various reagents to add either or both reactive groups and ionizable groups, or convert thiol reactive groups to ionizable groups. In embodiments, a polymer that possesses thiol reactive groups or other suitable reactive groups, such as anhydride groups, can be attached to the substrate, where the substrate reacts with the reactive groups and produces an attachment group -A-, an ionizable group, or both. In embodiments, the polymer having thiol reactive groups can be generated on the surface of the substrate *in-situ* (i.e., monomers can be polymerized on the substrate surface) to afford the surface polymer having **-S-H** reactive

groups, or subsequently modified to have **-S- H** reactive groups.

In embodiments, the polymer layer can have a dry thickness of, for example, from about 5 to about 1,000 nanometers, including intermediate values and ranges. In embodiments, the polymer on the surface can have a thickness in aqueous media of, for example, from about 50 to about 10,000 nanometers, including intermediate values and ranges. Advantageously, the polymer is a hydrogel.

In embodiments, the tie-layer attachment group **A** can be, for example, a carboxyl group attached to a tie-layer on the substrate surface.

In embodiments, the polymer can be, for example, a poly(alkylene-alt-maleic anhydride) first modified with an alkyl-amine compound and then modified with a thiol-alkyl-amine compound to provide a thiol presenting surface. The disclosed polymer can comprise, for example, a mixture of maleic anhydride and alkylene monomers in a mole ratio of about 1.0: 1.5 to about 1.5: 1.0, including intermediate values and ranges.

In embodiments, the thiol-modified surface can be, for example, a carboxy polymer surface comprising a poly (ethylene-alt-maleic anhydride) having from about 30 to about 50 mol% of the initial anhydride groups reacted with propylamine, including intermediate values and ranges. In embodiments, the polymer can comprise a maleic anhydride copolymer modified with cystamine, or like compounds.

In embodiments, the polymer can comprise a plurality of reactive thiol groups and a plurality of ionizable carboxyl- and amino-groups, and a salt thereof, a combination thereof or a combination of a non-salt and a salt.

In embodiments, the tie-layer comprises a condensable or co-polymerizable silane compound. If a preformed anhydride containing polymer is selected, such as a dEMA polymer, then the tie-layer can be, for example, an amino-alkyl silane such as amino-propyl silane (APS). If an *in- situ* formed polyacrylate type polymer is selected for the polymer layer, then a vinyl functionalized silane suitable for copolymerization with the acrylate monomer(s), such as a methacryloyloxypropyltrimethoxysilane (MOPS), can be selected as the silane for the tie layer.

In embodiments, the disclosure provides a method to immobilize a protein, comprising:

combining the protein and a mixture comprised of an activated linker compound having a maleimide group in a buffer solution, to form a maleimide-modified protein; and

contacting the maleimide-linker-modified protein and a buffer swollen, thiol-modified, surface bound, polymer of the disclosure to immobilize the maleimide-linker-modified protein on the polymer surface.

In forming the maleimide-modified protein, the protein and the activated linker compound can be present, for example, in a relative mole ratio of from about 1:1 to about 50:1, including intermediate values and ranges. The maleimide-modified protein can be, for example, immobilized at from about 300 to about 5,000 picometers (pm) including intermediate values and ranges.

In embodiments, the method can further comprise contacting the surface immobilized protein with a ligand. The method can also further comprise detecting ligand binding by the surface immobilized protein. The detected ligand binding by the surface immobilized protein can be, for example, from about 40 to about 5,000 picometers (pm), including intermediate values and ranges.

In embodiments, a particularly useful molar ratio of protein-to-linker (P:L) for immobilization is from about 1: 2 to about 1:100, including intermediate values and ranges, and the a particularly useful molar ratio of protein-to-linker (P: L) for binding is from about 1:10 to about 1:30, including intermediate values and ranges.

In embodiments, the Z' for immobilizing the protein to the surface can be, for example, of from about 0.3 to about 1.0, and from about 0.3 to about 0.6, including intermediate values and ranges.

In embodiments, the thiol-modified surface can be, for example, a carboxy polymer surface comprising at least one of:

an alkyl-amine modified poly(alkylene-alt-maleic anhydride);
a poly(acrylic acid); or
a copoly(acrylic acid-acrylamide),

the polymer having a plurality of reactive groups and a plurality of ionizable groups, or a combination of two or more of the polymers, and a salt thereof, or a combination of a non-salt and a salt. Preferably, the plurality of reactive groups are **-S- H-** reactive groups capable of reacting with a maleimide-modified target. Advantageously, at least one of the reactive groups has a spacer unit situated between the polymer backbone and the reactive group.

In embodiments, the spacer unit situated between the polymer backbone and the reactive group is selected from a branched or unbranched, substituted or unsubstituted ($C_{1-20}$) hydrocarbylene, or an oxyhydrocarbylene glycol of the formula $-CHR^3-CHR^3-(O-CHR^3-CHR^3)_z-$, or a combination thereof, $\mathbf{R^3}$ is **H,** or a branched or unbranched, substituted or unsubstituted ($C_{1-8}$)alkyl, and z is an integer of from 1 to about 10.

In embodiments, the disclosed polymeric coated substrate surface can be used, for example, for forming a device or material for use in cell culture, purification of materials from cell culture, a bioassay, implantation in a patient as a stent, catheter, prosthetic, implant, or graft, and an analytical or a sensing device.

In embodiments, the thiol-modified surface can be, for example, a carboxy polymer surface comprising a poly (ethylene-alt-maleic anhydride) having from about 30 to about 50 mol% of the initial anhydride groups reacted with propylamine. In embodiments, the protein can be present, for example, in an amount of from about 10 micrograms/mL to about 100 micrograms/mL, and the maleimide-linker-modified protein can be immobilized on the substrate, for example, at an indication of from about 400 to about 5,000 picometers as measured by a resonance wave guide biosensor.

In embodiments, the immobilization method can further comprise rinsing the protein contacted substrate with buffer to remove free protein, rinsing the ligand contacted substrate with buffer to remove free ligand, or a combination thereof.

In embodiments, the disclosure provides a thiol-modified surface article prepared by the process comprising:

contacting a carboxy polymer surface with a protected-thiol source reagent to form a protected disulfide group, and deprotecting the protected disulfide group.

In embodiments, the disclosure provides an article comprising the thiol-modified surface for immobilization. The article can further comprise at least one protein immobilized on the surface.

Referring to the Figures, Fig. 1 shows a generalized SH-maleimide coupling reaction where the maleimide-modified target (MAL-R') is a Micheal acceptor for the HS-R" nucleophile where R" represents the thiol-modified surface and R' represents the maleimide-linker-modified protein. Fig. 2 schematically shows a first step of the functionalization of a protein with a maleimide-linker compound, and a second step where the maleimide-linker functionalization protein is subsequently and specifically bound to an **-SH** bearing surface presumably via the Micheal reaction of Fig. 1.

Fig. 3 schematically illustrates a preparative sequence for forming a thiol-modified polymer surface on a substrate, for example, corresponding to structural formula **(IIIa).** A suitable substrate is first contacted with an amino-silane to form, for example, an APS (amino-propyl-silane) coated surface **(1).** Next an EMA polymer or a dEMA polymer or like polymer (i) can be bound to, associated with, or generated *in situ*, to provide the dEMA polymer or like polymer fixed to the amino-silane(2). The EMA polymer or dEMA polymer can optionally be derivatized with, for example, an amine compound, such as propyl amine, prior to (i.e., pre-blocked) or after the time when the EMA polymer or dEMA polymer is on the surface. The EMA polymer or dEMA polymer surface can be reacted with, for example, cystamine or like compound (ii) to form the amino-disulfide derivative **(3).** The amino-disulfide derivative can then be reduced (iii) with any suitable reducing agent including, for example, DL-dithiothreitol (DTT), 1,4-dithioerythritol (DTE), ammonium thioglycolate, (tris(2-carboxyethyl)phosphine hydrochloride) (TCEP), and like agents, or mixtures thereof, resulting in the desired thiol surface **(4).**

Fig. 4 illustrates exemplary carbonic anhydrase (CAII) immobilization on the dEMA-thiol-modified surface, for example, corresponding to structural formula **(IIIa),** at 100 microg/mL overnight at 4°C in acetate buffer (20 mM; pH 5.5), in the decreasing ratio of MAL-AHA-NHS/CAII indicated, that is the relative amount of MAL-AHA-NHS reagent used to modify the CAII protein target prior to contacting with a thiol-modified surface for immobilization. The observed beta signal atop the bars is in picometers. See Example 1.

Fig. 5 illustrates exemplary furosemide binding on thiol-surface immobilized maleimide-modified carbonic anhydrase (CAII) overnight at 4°C in acetate buffer (20 mM; pH5.5). The thiol-surface selected was a (PAA)-thiol modified surface and the binding was measured as a function of various concentrations of thiol surface modifying reagents or precursors, such as cystamine, cystine, or cystamine and glycine combinations.

Fig. 6 illustrates exemplary carbonic anhydrase (CAII) immobilization on a thiol surface corresponding to structural formula **(IVa).**

Fig. 7 illustrates furosemide binding on carbonic anhydrase (CAII) that has been immobilized on a thiol-modified surface according to structural formula **(IVa).**

Fig. 8 illustrates exemplary carbonic anhydrase (CAII) immobilized on a thiol surface polymer corresponding to structural formula **(IVa).** The CAII was immobilized overnight at 4°C in acetate buffer (20 mM; pH 5.5) on a co-poly (acrylic acid - methacrylamide)(PAA/MAm)-thiol modified surface as a function of various concentrations of cystamine, or cystamine and glycine combinations.

Fig. 9 illustrates furosemide binding on immobilized carbonic anhydrase (CAII) as a function of various concentrations of CAII and as discussed in Example 3. The CAII was immobilized on a thiol surface polymer corresponding to structural formula **(IVa).**

## EXAMPLES

The following examples serve to more fully describe the manner of using the above-described disclosure, as

well as to set forth the best modes contemplated for carrying out various aspects of the disclosure. It is understood that these examples do not limit the scope of this disclosure, but rather are presented for illustrative purposes. The working examples further describe how to make and use the articles and methods of the disclosure.

The starting materials, such as synthetic, semi-synthetic, naturally occurring proteins or native proteins, and like materials, are commercially available such as from Sigma-Aldrich and like suppliers; can be, for example, prepared by known methods; can be isolated from a complex matrix by known methods, and like sources and methods. All commercially available chemicals were used as received.

Buffers

Acetate buffer (Fisher): 0.410 g sodium acetate in 250 mL ultra-pure water after which the pH is adjusted to 5.5 using 1M HCl and the solution is filtered.

Borate buffer: 3.81 g of $NaB(OH)_4$ is dissolved in ultra-pure water and the solution is filtered (pH 9.2).

PBS (Sigma): 1 tablet is dissolved in 200 mL of ultra-pure water and the solution is filtered (pH 7.5).

**Example 1**

**An Epic® 5041 sample plate surface was thiol-modified and an assay with a maleimide-modified or functionalized CAII accomplished.**

**Part 1: Method for Preparing the Thiol Surface** A thiol surface was prepared in a two-step procedure starting from a commercially available dEMA Epic® 5041 plate (Corning, Inc.) and as illustrated in Fig. 5. In a first step, cystamine was reacted with the maleic anhydride functionalities of the dEMA polymer matrix in basic media. In a second step the disulfide bonds of cystamine were reduced. The reduction can be carried out with any suitable reducing agent including DL-dithiothreitol (DTT), 1,4-dithioerythritol (DTE), ammonium thioglycolate, (tris(2-carboxyethyl)phosphine hydrochloride) (TCEP), and like agents, or mixtures thereof, including electrochemical and like methods. On microscope slides each of these reducing agents gave similar results when the reducing agent was added at about 50 to about 200 mM concentration and after for about 16 hr. In the microplate, where proper washing of the wells can be problematic, superior results were obtained using TCEP as the reducing agent. The reduced plate surface was not stored, as adjacent thiol groups can react to form new disulfides. Instead the plate was used directly for maleimide-modified-protein immobilization. A typical immobilization procedure is, for example:

Step 1: To each well of a commercial Epic® plate with dEMA surface was added 25 microliters of cystamine dihydrochloride (98%, Aldrich) dissolved in borate buffer at 200 mM. A series of 10 mixes at 10 microliters was performed and the plate was centrifuged at 800 rpm for 1 min. After 1 h the plate was rinsed (3 cycles of 25 microliters each) with water and the plate was centrifuged upside down to remove residual liquid (800 rpm, 1 min).

Step 2: To each well of the plate was added 15 microliters of TCEP (Sigma) reducing agent at 50 mM in water. 10 mixes of 10 microliters were performed and the plate was centrifuged at 800 rpm for 1 min. After 24 h at room temperature the plate was rinsed thoroughly with water, i.e., 3 times 3 rinses of 25 microliters of water each with a five times 15 microliter mixing cycle and an upside down centrifugation between each rinse.

**Part 2: Maleimide-modified or functionalized protein and immobilization of the maleimide-modified protein on a thiol-modified surface and a protein activity assay therewith** Maleimide groups were conveniently linked to a target protein via amine functionalities from basic amino acids such as lysine of the protein. In this example the protein was modified at 1:6 molar equivalents of protein to the maleimide-linker.

**Step 1. protein modification:** to 700 microliters of carbonic anhydrase solution (CAII, Sigma @ 1 mg/mL in water) was added 42 microliters of 6-maleimidohexanoic acid N-hydroxysuccinimide ester (98%, Aldrich, MAL-AHA-NHS at 1 mg/mL in water) (i.e., maleimide-linker reagent) and 6,258 microliters of acetate buffer. The solution was allowed to react for about 1.5 h. This maleimide-modified carbonic anhydrase was designated as MAL-AHA-CAII.

**Step 2. protein immobilization:** To a number of wells on a freshly prepared thiol surface of Example 1 was added 15 microliters of the abovementioned maleimide-modified protein solution. To the remaining wells 15 microliters of acetate buffer was added. The plate was centrifuged for 1 min at 800 rpm and kept at about +4 °C for about 16 hours.

**Step 3. binding protein inhibitor: The plate was equilibrated at ambient** (25°C) temperature for about 30 min before rinsing with PBS (on Biomek, following standard rinsing protocol). The plate was centrifuged and soaked in 0.1 % DMSO in PBS for about 4 h at ambient temperature.

A base line of the plate was obtained using the Epic® beta instrument for about 30 min, after which 15 microliters of furosemide solution (10 microM in PBS) or 0.1 % DMSO in PBS was added to the wells and mixed for about 3 min. The plate was then read in the Epic® instrument for 20 min.

## Part 3: Results and Discussion

**Immobilization of maleimide-linker modified carbonic anhydrase; Maleimide/carbonic anhydrase linker to protein ratio:** An optimum protein:linker ratio can be determined where the amount of maleimide-linker reagent, such as 6-maleimidohexanoic acid N-hydroxysuccinimide ester (MAL-AHA-NHS) linker, is varied relative to the protein. Using the known molecular weights, the molar ratio expressed in equivalents of linker to protein, can be calculated. For maleimide-linker modified-protein immobilizations, there is an apparent steep immobilization increase from 0 to about 12 equivalents of maleimide-linker reagent to protein, after which immobilization levels off to about 100 equivalents at about 4,500 picometers (pm). In contrast, for ligand binding to the immobilized maleimide-linker modified-protein there appears to be a maxima with from about 10 to about 20 equivalents of maleimide-linker reagent to protein to just over about 20 pm. After this maxima, additional maleimide-linker apparently impairs the protein function, and the observed binding signal decreases. The immobilization and binding results on thiol-modified polymer surface are summarized in Table 2.

**Table 2. Summary of immobilization and binding results on thiol- modified polymer surfaces.**

| MALAHANHS/ CAII ratio | Protein Immob. level (pm) | Immob. SD (+)[1] | Observed binding level (pm) | Binding SD (+)[1] | Availability % | Z' |
|---|---|---|---|---|---|---|
| 96 | 4506 | 357 | 20.07 | 1.72 | 20 | 0.51 |
| 48 | 4612 | 334 | 21.94 | 1.50 | 22 | 0.63 |
| 24 | 4520 | 340 | 23.19 | 1.65 | 23 | 0.55 |
| 12 | 4075 | 274 | 22.00 | 1.67 | 25 | 0.58 |
| 6 | 3248 | 188 | 16.58 | 1.55 | 23 | 0.37 |
| 3 | 2177 | 179 | 9.51 | 1.56 | 20 | 0.12 |
| 0 | 196 | 78 | -0.41 | 1.12 | -10 | **-N.A[4].-** |

1. Immob. SD(+) or Binding SD(+) refer to standard deviation of the positive wells.
2. Availability% is calculated by the formula binding observed/max binding *100 where max binding is defined as immobilization level*($M_w$ drug/$M_w$ protein)*(RII drug/RII protein)*stoichiometry of drug/protein binding event
3. Z' is calculated by the formula Z' = 1-((3*(SD(+) + SD(-)))/(binding observed)
4. N.A.- Not applicable since no immobilization.

**Table 3. Summary of immobilization and binding results on thiol-modified polymer surfaces.**

| Carbonic Anhydrase Concentration (CAII) (micrograms/ mL) | Protein immob. level (pm) | Immob. SD(+) | Binding observed | Binding SD(+) | Availability % | Z' |
|---|---|---|---|---|---|---|
| 100 | 3174 | 174 | 17.20 | 2.29 | 25 | 0.31 |
| 75 | 2083 | 286 | 10.88 | 1.97 | 24 | 0.15 |
| 50 | 1263 | 172 | 4.86 | 1.41 | 18 | -0.61 |
| 25 | 514 | 81 | 0.94 | 0.90 | 8 | -4.80 |
| 10 | 258 | 73 | -0.12 | 0.84 | -2 | 4.798 |

## Example 2

**An Epic® 5046 sample plate surface was thiol-modified and an assay with a maleimide-modified CAII or functionalized CAII accomplished.**

**Part 1: Method for Preparing the Thiol Surface** A thiol surface was prepared in a two-step procedure starting from a commercially available Epic® 5046 plate (Corning, Inc.) to give a polymer of structural formula **IV**. To ensure a negative charge on the resulting surface, cystine, instead of cystamine used in Example 1, was employed. In a second step the disulfide bonds of cystamine were reduced. The reduction can be carried out with any suitable reducing agent or mixtures thereof, including electrochemical methods. The reduced plate surface was used directly for maleimide-modified-protein immobilization. A typical immobilization procedure is, for example:

**Step 1:** To each well of a commercial Epic® plate with dEMA surface was added 25 microliters of borate buffer saturated with cystine (Acros). A series of 10 mixes at 10 microliters was performed and the plate was centrifuged at 800 rpm for 1 min. After 1 hr the plate was rinsed (3 cycles of 25 microliters each) with water and the plate was centrifuged upside down to remove residual liquid (800 rpm, 1 min).

**Step 2:** To each well of the plate was added 25 microliters of DTT (Sigma) reducing agent at 200 mM in water. 10 mixes of 10 microliters were performed and the plate was centrifuged at 800 rpm for 1 min. After 24 h at room temperature the plate was rinsed thoroughly with water, i.e., 3 times 3 rinses of 25 microliters of water each with a five times 15 microliter mixing cycle and an upside down centrifugation between each rinse.

**Part 2: Maleimide-modified or functionalized protein and immobilization of the maleimide-modified protein on a thiol-modified surface and a protein activity assay therewith** Maleimide groups were conveniently linked to a target protein via amine functionalities from basic amino acids such as lysine of the protein. In this example the protein was modified at four(4) different ratios, of about 1:24, 1:12, 1:6, and 1:3 molar equivalents of protein to the maleimide-linker. Unmodified protein was used as reference.

Step **1. protein modification:** to 240 microliters of carbonic anhydrase solution (CAII, Sigma @ 1 mg/mL in water) was added 61, 30.5, 15.3 or 7.6 microliters of 6-maleimidohexanoic acid N-hydroxysuccinimide ester (98%, Aldrich, MAL-AHA-NHS at 1 mg/mL in water) (i.e., maleimide-linker reagent). The solution was allowed to react for about 1.5 h and acetate buffer to make the total volumes 2,500 microliter.

**Step 2. protein immobilization:** To a number of wells on a freshly prepared thiol surface of Example 1 was added 15 microliters of the abovementioned maleimide-modified protein solutions. To the remaining wells 15 microliters of acetate buffer was added. The plate was centrifuged for 1 min at 800 rpm and kept at about +4 °C for about 16 hours.

**Step 3. binding protein inhibitor:** The plate was equilibrated at ambient (25°C) temperature for about 30 min before rinsing with PBS (on Biomek, following standard rinsing protocol). The plate was centrifuged and soaked in 0.1 % DMSO in PBS for about 4 h at ambient temperature.

A base line of the plate was obtained using the Epic® beta instrument for about 30 min, after which 15 microliters of furosemide solution (10 microM in PBS) or 0.1 % DMSO in PBS was added to the wells and mixed for about 3 min. The plate was then read in the Epic® instrument for 20 min.

### Part 3: Results and Discussion

**Immobilization of maleimide-linker modified carbonic anhydrase; Maleimide/carbonic anhydrase linker to protein ratio:** An optimum protein linker ratio can be determined where the amount of maleimide-linker reagent, such as 6-maleimidohexanoic acid N-hydroxysuccinimide ester (MAL-AHA-NHS) linker, is varied relative to the protein. Using the known molecular weights, the molar ratio expressed in equivalents of linker to protein, can be calculated. For maleimide-linker modified-protein immobilizations, there is a steep increase in immobilization and binding as the ratio increases from 0 to about 24 equivalents of maleimide-linker reagent to protein. At this level the immobilization is at about 2,000 picometers (pm) in relative units. The same trend is observed in the furosemide binding, where binding increases from 0 to about 16 pm. The immobilization and binding results on the thiol-modified polymer surfaces are summarized in Table 4.

**Table 4. Summary of protein immobilization and ligand binding results on thiol-modified polymer surfaces.**

| Ratio of Carbonic Anhydrase (CAII) : Maleimide linker | Protein immob- level (pm) | Binding observed | Binding SD(+) |
|---|---|---|---|
| 24 | 2149 | 15.69 | -0.14 |

(continued)

| Ratio of Carbonic Anhydrase (CAII) : Maleimide linker | Protein immob- level (pm) | Binding observed | Binding SD(+) |
|---|---|---|---|
| 12 | 1522 | 11.70 | -0.08 |
| 6 | 937 | 6.97 | -1.17 |
| 3 | 604 | 3.09 | -1.77 |
| 0 | 189 | -0.52 | 13.51 |

Example 3 (ACTUAL)

**A thiol-modified surface was prepared on an Epic® sample well plate having a surface bound copolymer of acrylic acid and acrylamide (AA/MAm) and an immobilization and binding assay with maleimide-modified CAII was accomplished.**

**Part 1: Surface Preparation on Copolymer (AA/MAm) surface** In this example the thiol functionality was bound to a surface made up of s-NHS activated carboxylic acid groups. To ensure a negative charge in the resulting thiol-modified surface, cystamine can be mixed with an amine or amino acid capable of introducing a negative charge, such as, for example glycine. Alternatively, cystine which can introduce both a thiol group and a negative charge can be used. The preparation of the poly(acrylic acid) or copoly(acrylic acid-acrylamide) can be prepared, for example, according to EP 0226470 (to Unilever) or copending EP08305845 (to Corning).

**Step 1:** To well column groupings 1 to 6 of a copolymeric (AA/MAm) PAA/PAAm microplate was added 15 microliters of one of the following solutions:

1) cystamine 200 mM (columns 1 to 4)
2) cystamine 50 mM (columns 5 to 8)
3) saturated cystine < 50 mM (columns 9 to 12)
4) cystine 10 mM (columns 13 to 16)
5) cystamine/glycine 100/100 mM (columns 17 to 20)
6) cystamine/glycine 25/25 mM (columns 21 to 24)

A series of 10 mixes at 10 microliters was performed and the plate was centrifuged at 800 rpm for 1 min. After 1 hr the plate was rinsed (3 cycles of 25 microliters each) and the plate was centrifuged upside down to remove all liquid (800 rpm, 1 min).

**Step 2:** To each well of the plate was added 15 microliters of TCEP (Sigma) reducing agent at 50 mM in water. 10 mixes of 10 microliters were performed and the plate was centrifuged at 800 rpm for 1 min. After 24 h at room temperature, the plate was rinsed thoroughly with water, i.e., 3 times 3 rinses of 25 microliters of water each with a five times 15 microliter mixing cycle and an upside down centrifugation step between each rinse.

**Part 2: Maleimide-modified protein; Immobilization of a maleimide-modified protein on a thiol-modified polymer surface; and a protein activity assay** Maleimide groups can be conveniently linked to a protein via amine functionalities from basic amino acids such as lysine of the protein. In this example the protein was modified at 1:6 molar equivalents of protein to maleimide-linker reagent.

**Step 1. Protein modification:** to 700 microliters of carbonic anhydrase solution (CAII, Sigma @ 1 mg/mL in water) was added 42 microliters of 6-maleimidohexanoic acid N-hydroxysuccinimide ester (98%, Aldrich, MAL-AHA-NHS @ 1 mg/mL in water) and 6,258 microliters of acetate buffer. The solution was allowed to react for about 1.5 h.

**Step 2. Protein immobilization:** To a number of wells on a freshly prepared thiol-modified polymer surface of Example 1 was added 15 microliters of the abovementioned maleimide-modified protein solution. To the remaining wells 15 microliters of acetate buffer was added. The plate was centrifuged for 1 min at 800 rpm and kept at about +4 °C for about 16 hours.

**Step 3. Binding of protein inhibitor:** The plate was equilibrated at ambient (25°C) temperature for about 30 min before rinsing with PBS (on Biomek, following standard rinsing protocol). The plate was centrifuged and soaked in 0.1 % DMSO in PBS for about 4 h at ambient temperature.

A base line of the plate was obtained using the Epic® beta instrument for about 30 min, after which 15 microliters of furosemide (protein inhibitor ligand) solution (10 microM in PBS) or 0.1 % DMSO in PBS was added to the wells and mixing carried out for about 3 min. The plate was then read in the Epic® instrument for 20 min.

**Part 3: Results:** The immobilization data shows that the introduction of a negative charge has a favorable

impact on the amount of protein immobilized. On a thiol-modified polymer surface that has been derivatized with cystine or a cystamine/glycine mixture charge modifying agents, a signal between 2,500 and 3,500 pm was observed for protein immobilization. In contrast, for a neutral surface the signal was only about 2,300 pm, corresponding to around 30 % less immobilized protein.

**Table 5. Summary of immobilization results accomplished with cystine or a cystamine/glycine charge modifiers on thiol-modified polymer surfaces.**

| Charge Modifier group ID | Positive Wells (x) | Positive Wells (s) | Negative Wells (x) | Negative Wells (s) | Total Immob. (RU) |
|---|---|---|---|---|---|
| cystamine 200 mM | 833,087.28 | 384.78 | 830,751.43 | 401.32 | 2,335.86 |
| cystamine 50 mM | 832,882.40 | 593.33 | 830,627.57 | 556.40 | 2,254.83 |
| saturated cystine | 833,925.64 | 681.41 | 830,667.48 | 362.42 | 3,258.16 |
| cystine 10 mM | 833,494.19 | 853.04 | 830,686.84 | 480.51 | 2,807.35 |
| cystamine/ glycine 100/100 mM | 832,971.72 | 686.87 | 830,279.15 | 395.07 | 2,692.57 |
| cystamine/ glycine 25/25 mM | 833,091.13 | 613.54 | 830,368.42 | 461.70 | 2,722.71 |

The differences in the observed binding realized for the different reaction conditions were further demonstrated. When the thiol-modified surface was further derivatized with cystine prior to ligand binding, a binding of 25 pm can be observed. When cystine in combination with glycine was used a binding value of just over 10 pm was observed. For the experiment where the thiol-modified surface no longer carries a negative charge, 10 pm or less was observed. This experiment shows that choice of the reagent used to transform an activated ester sNHS containing surface into a thiol-modified surface was significant for the use of the surface in an assay.

**Table 6. Summary of binding results accomplished with cystine or a cystamine/glycine charge modifiers on thiol-modified polymer surfaces.**

| Grp No. | Group Identification | Total Signal (X)[1] | Activity %[2] | Z'[3] |
|---|---|---|---|---|
| 1 | cystamine 200 mM | 8.19 | 28.77 | 0.08 |
| 2 | cystamine 50 mM | 11.31 | 23.35 | 0.01 |
| 3 | saturated cystine | 25.70 | 22.38 | 0.08 |
| 4 | cystine 10 mM | 24.04 | 26.16 | 0.01 |
| 5 | cystamine/glycine 100/100 mM | 12.66 | 26.71 | 0.07 |
| 6 | cystamine/glycine 25/25 mM | 12.95 | 27.35 | 0.16 |

1. Total Signal (x) Epic® signal observed calculated as the difference between signal in wells containing immobilized protein and the wells not containing protein.
2. Activity % is defined above.
3. Z' is defined above.

The disclosure has been described with reference to various specific embodiments and techniques. However, it should be understood that many variations and modifications are possible while remaining within the spirit and scope of the disclosure.

## Claims

**1.** A label-free biosensor article comprising:

a substrate having a tie-layer attached to the substrate surface, and
a polymer attached to the tie-layer, the attached polymer being a thiol-modified surface comprised of mers of the formula **(I):**

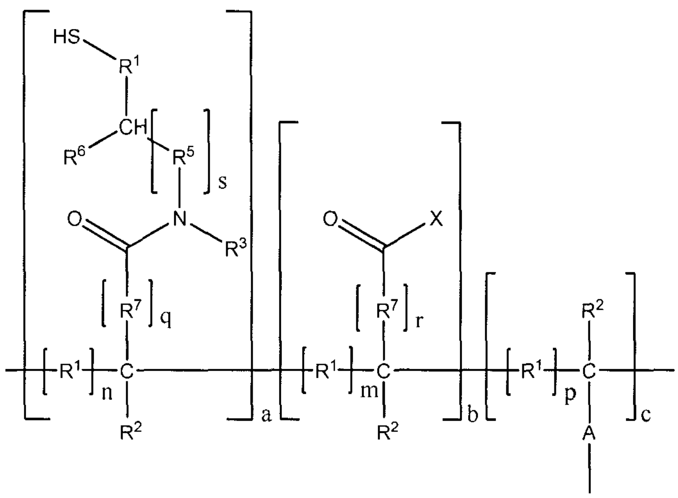

**(I)**

where
**A** is a tie-layer attachment group;
***a*** comprises a thiol containing mer, the thiol being suitable to immobilize a maleimide-modified bioentity;
***b*** comprises an amide or a carboxylic acid containing mer;
***c*** comprises at least one mer having the tie-layer attachment group **A;**
***a***, ***b***, and **c** are each independently an integer from 1 to about 100,000;
***m***, ***n***, ***p***, ***q***, ***r***, and ***s***, are each independently 0 or 1;
**X** is $-N(R^3)(R^4)$, or $-OR^3$;
each $\mathbf{R^1}$ is independently a divalent $(C_{1-4})$hydrocarbyl;
each $\mathbf{R^2}$ is independently -H or monovalent $(C_{1-4})$hydrocarbyl;
$\mathbf{R^3}$ and $\mathbf{R^4}$ are each independently -H, or a monovalent substituted or unsubstituted $(C_{1-10})$hydrocarbyl;
$\mathbf{R^5}$ is a divalent alkyl amide or alkoxylate amide spacer of the formulas $-(CH_2)_u-C(O)N(R^3)-$ or $-(CH_2CH_2O)_u-C(O)N(R^3)-$, where u is an integer from 1 to 10;
$\mathbf{R^6}$ is independently -H or -COOH; and
each $\mathbf{R^7}$ is independently a carbon-carbon bond or a divalent substituted or unsubstituted $(C_{1-4})$ hydrocarbyl, and a salt thereof, or a combination of a non-salt and a salt,
or mers of the formula **(II)**:

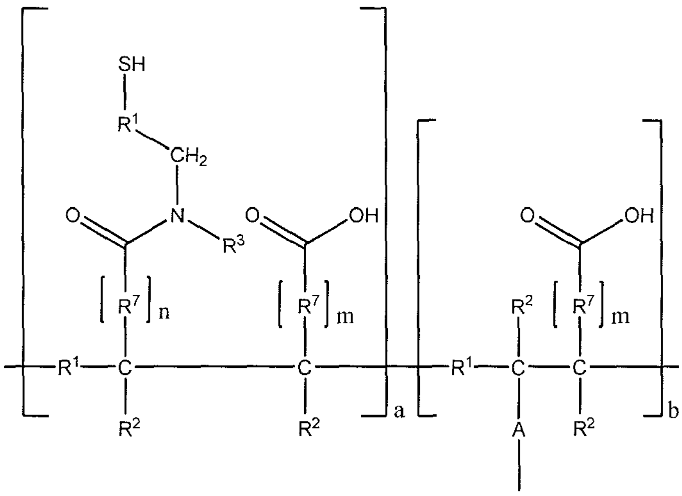

**(II)**

where

***a*** comprises a half-thiol half-acid containing mer, the thiol being suitable to immobilize a maleimide-modified bioentity;

***b*** comprises at least one mer having a tie-layer attachment group **A;**

***a*** and ***b*** are each independently integers from 1 to about 100,000;

***n*** and ***m*** are each independently either 0 or 1;

$\mathbf{R^1}$ is a divalent substituted or unsubstituted $(C_{1-10})$hydrocarbyl;

$\mathbf{R^2}$ is a H or $(C_{1-4})$hydrocarbyl;

$\mathbf{R^3}$ is -H or a monovalent substituted or unsubstituted $(C_{1-10})$hydrocarbyl; and

$\mathbf{R^7}$ is a carbon-carbon bond or a divalent substituted or unsubstituted $(C_{1-4})$ hydrocarbyl,

and a salt thereof, or a combination of a non-salt and a salt,

or mers of the formula **(III):**

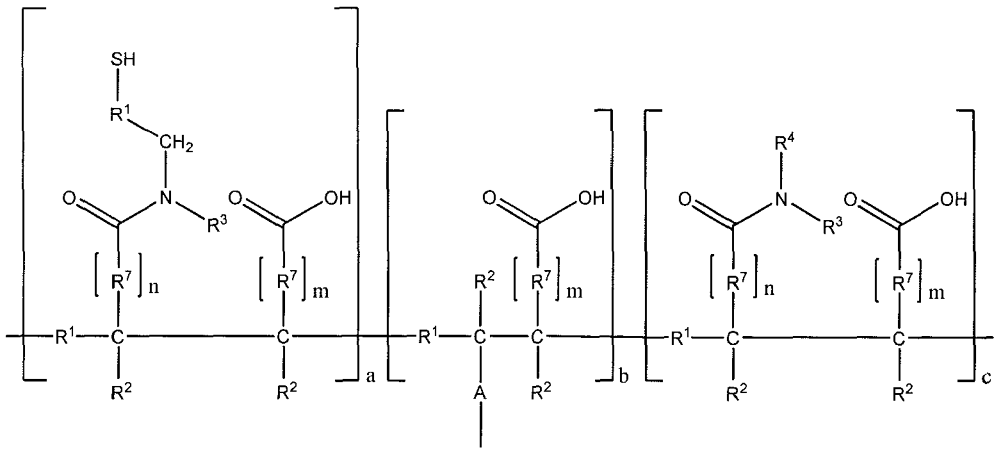

**(III)**

where

***a*** comprises a half-thiol half-acid containing mer, the thiol being suitable to immobilize a maleimide-modified bioentity;

***b*** comprises at least one mer having a tie-layer attachment group **A;**

***c*** comprises a half-amide half-acid containing mer;

***a***, ***b***, and c are each independently an integer from 1 to about 100,000;

***n*** and ***m*** are each independently 0 or 1;

$\mathbf{R^1}$ is a divalent substituted or unsubstituted $(C_{1-10})$hydrocarbyl;

$\mathbf{R^2}$ is a H or $(C_{1-4})$hydrocarbyl;

$R^3$ and $R^4$ are each independently -H or a monovalent substituted or unsubstituted $(C_{1-10})$hydrocarbyl; and
$R^7$ is a carbon-carbon bond or substituted or unsubstituted $(C_{1-4})$ hydrocarbyl, and a salt thereof, or a combination of a non-salt and a salt.

**2.** The article of claim 1 wherein the polymer layer has a dry thickness of from about 5 to about 1,000 nanometers.

**3.** The article of claim 1 wherein the polymer attached to the surface has a thickness in aqueous media of from about 50 to about 10,000 nanometers.

**4.** The article of claim 3 wherein the polymer is a hydrogel.

**5.** The article of any one of claims 1 to 4 wherein the tie-layer attachment group **A** is a carboxyl group attached to the tie-layer of the substrate surface.

**6.** The article of any one of claims 1 to 5 wherein the polymer comprises a poly(alkylene-alt-maleic anhydride) modified with an alkyl-amine compound and further modified with a thiol-alkyl-amine compound to provide a thiol modified surface.

**7.** The article of any one of claims 1 to 6, wherein the thiol-modified surface comprises a carboxy polymer surface comprising a poly (ethylene-alt-maleic anhydride) having from about 30 to about 50 mol% of the initial anhydride groups reacted with an alkylamine, and from about 50 to about 70 mol% of the residual anhydride groups reacted with a thioalkylamine.

**8.** The article of any one of claims 1 to 7 wherein the polymer comprises a plurality of reactive thiol groups and a plurality of ionizable carboxyl and amino groups, and a salt thereof, or a combination of a non-salt and a salt.

**9.** The article of claim 1 wherein the tie-layer comprises a condensable or co-polymerizable silane compound.

**10.** A method to immobilize a protein, comprising:

combining the protein with a mixture comprised of an activated linker compound having a maleimide group in a buffer solution, to form a maleimide-linker-modified protein; and
contacting the maleimide-linker-modified protein and a buffer swollen, thiol-modified, surface bound, polymer of claim 1 to immobilize the maleimide-linker-modified protein on the polymer surface.

**11.** The method of claim 10, further comprising contacting the surface immobilized protein with a ligand.

**12.** The method of claim 11, further comprising detecting ligand binding by the surface immobilized protein.

**13.** The method of claim 12, wherein the detected ligand binding by the surface immobilized protein is from about 1 to about 1,000 picometers (pm).

**14.** The method of any one of claims 10 to 13, wherein the protein is immobilized at from about 300 to about 5,000 picometers (pm).

**15.** The method of any one of claims 10 to 14, wherein the protein and the activated linker compound are in a relative mole ratio of from about 1:1 to about 50:1.

**16.** The method of any one of claims 10 to 15, wherein the activated linker compound comprises at least one compound of the formula:

$$\mathbf{B\text{-}R^1\text{-}c}$$

where
**B** comprises a monovalent Micheal acceptor group, which reacts second with the thiol-modified surface;
**C** comprises a monovalent reactive group, which reacts first with an amine of the protein; and
**-R¹-** is a divalent spacer group selected from a branched or unbranched, substituted or unsubstituted $(C_{1-20})$hydro-

carbylene, or an oxyhydrocarbylene glycol of the formula $-CHR^3-CHR^3-(O-CHR^3-CHR^3)_z-$, or a combination thereof, $\mathbf{R^3}$ is **H,** or a branched or unbranched, substituted or unsubstituted $(C_{1-8})$alkyl, and *z* is an integer of from 1 to about 10.

**17.** The method of claim 16, wherein **B** is a maleimide group, **C** is a succinimidyl ester group, and $\mathbf{-R^1-}$ is a branched or unbranched, substituted or unsubstituted $(C_1\text{-}_{20})$hydrocarbylene.

**18.** The method of any one of claims 10 to 17, wherein the molar ratio of protein-to-linker (P:L) for immobilization is from about 1: 2 to about 1:100 and the molar ratio of protein-to-linker (P:L) for binding is from about 1:10 to about 1:30.

**19.** The method of any one of claims 10 to 18, wherein the thiol-modified surface comprises a carboxy polymer surface comprising at least one of:

an alkyl-amine modified poly(alkylene-alt-maleic anhydride);
a poly(acrylic acid); or
a copoly(acrylic acid-acrylamide),
the polymer having a plurality of **-S- H** reactive groups capable of reacting with a maleimide-modified target, and a plurality of ionizable groups, or a combination of two or more of the polymers, and a salt thereof, or a combination of a non-salt and a salt.

**20.** The method of claim 19, wherein at least one of the reactive groups of the carboxy polymer surface has a spacer unit situated between the polymer backbone and the reactive group.

**21.** The method of claim 20, wherein the spacer unit situated between the polymer backbone and the reactive group is selected from a branched or unbranched, substituted or unsubstituted $(C_{1-20})$ hydrocarbylene, or an oxyhydrocarbylene glycol of the formula $-CHR^3-CHR^3-(O-CHR^3-CHR^3)_z-$, or a combination thereof, $\mathbf{R^3}$ is **H,** or a branched or unbranched, substituted or unsubstituted $(C_{1-8})$alkyl, and z is an integer of from 1 to about 10.

FIG. 1

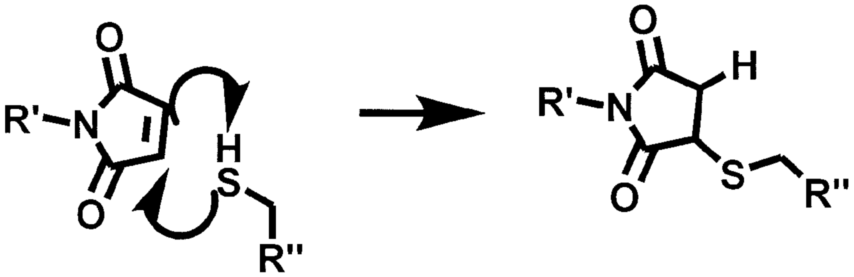

FIG. 2

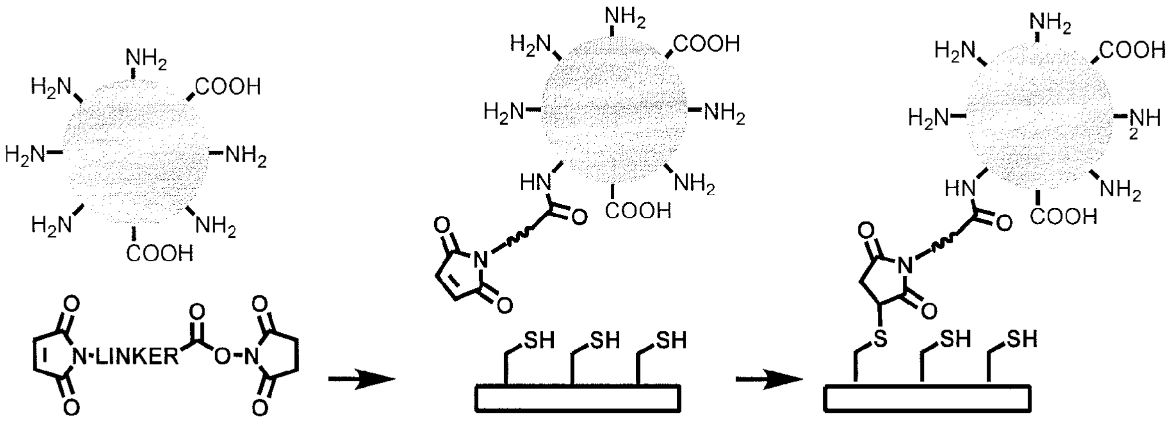

# FIG. 3

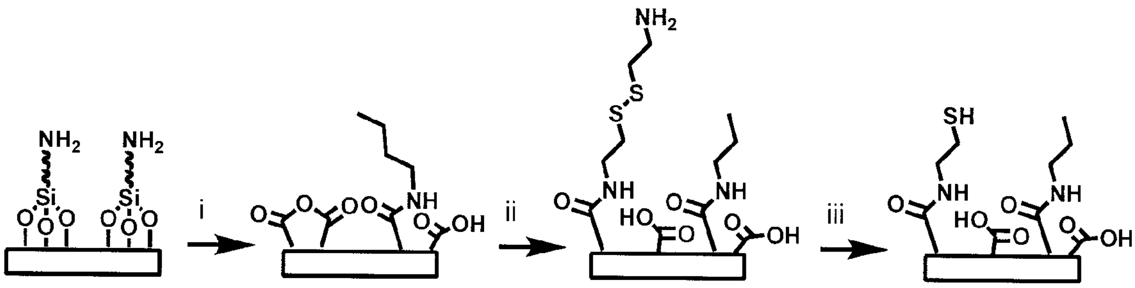


1 2 3 4

# FIG. 4

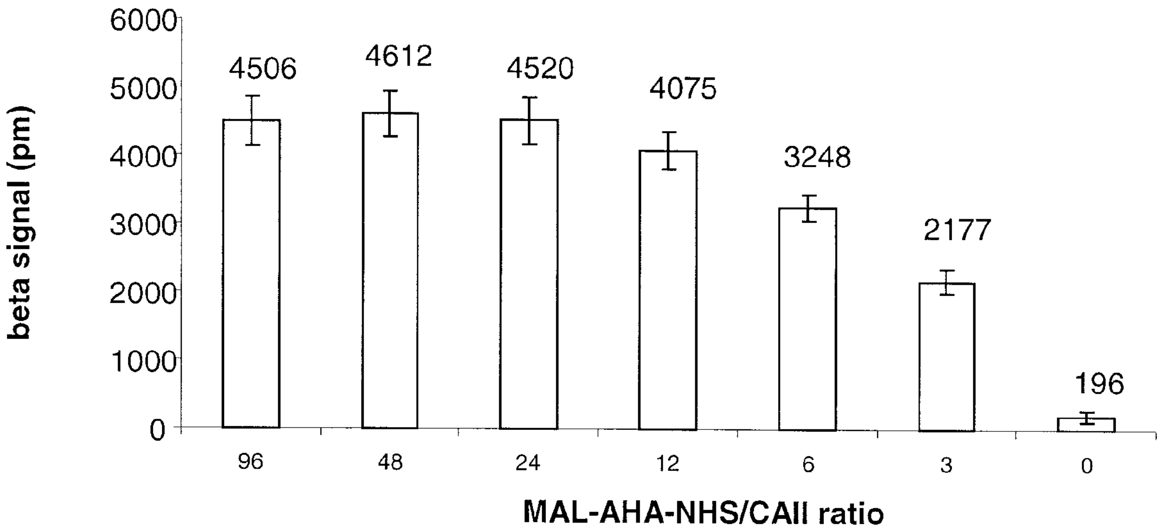

# FIG. 5

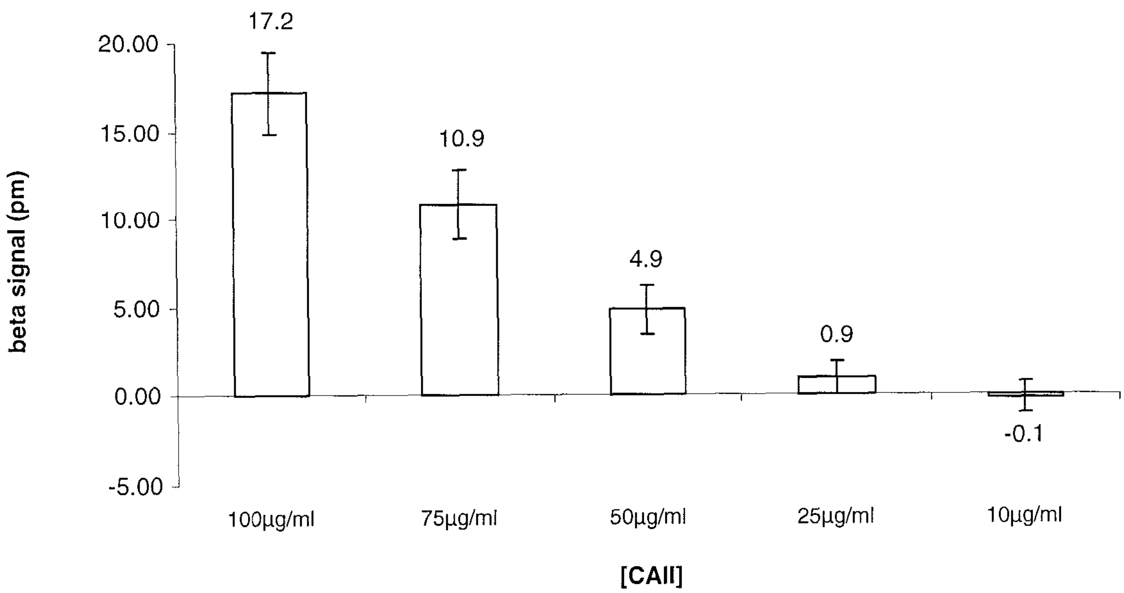
17.2
10.9
4.9
0.9
-0.1
20.00
15.00
10.00
5.00
0.00
-5.00
beta signal (pm)
100µg/ml
75µg/ml
50µg/ml
25µg/ml
10µg/ml
[CAII]

# FIG. 6

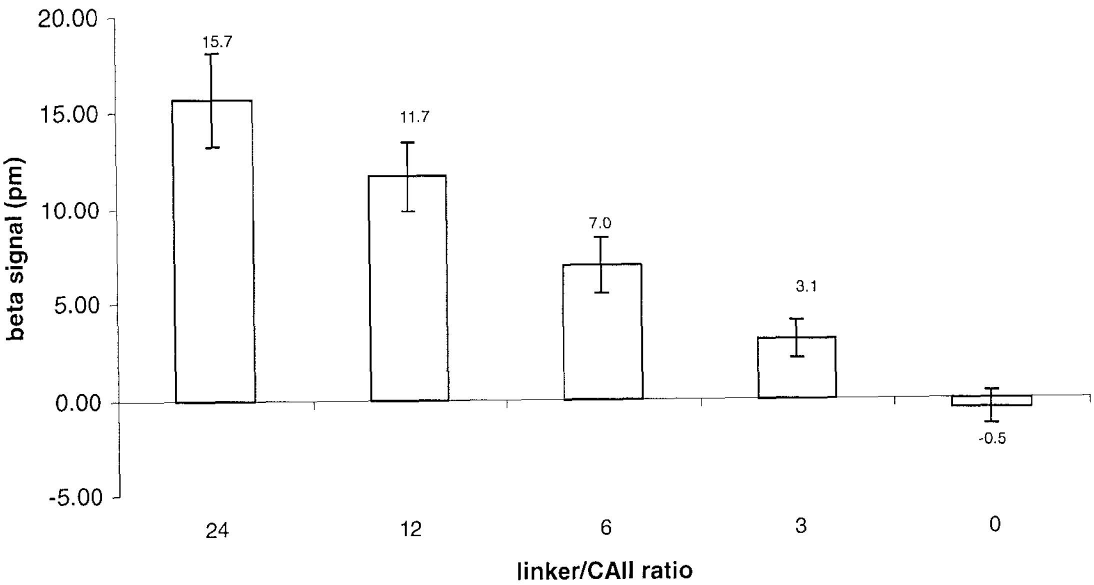
15.7
11.7
7.0
3.1
-0.5
20.00
15.00
10.00
5.00
0.00
-5.00
beta signal (pm)
24
12
6
3
0
linker/CAII ratio

# FIG. 7

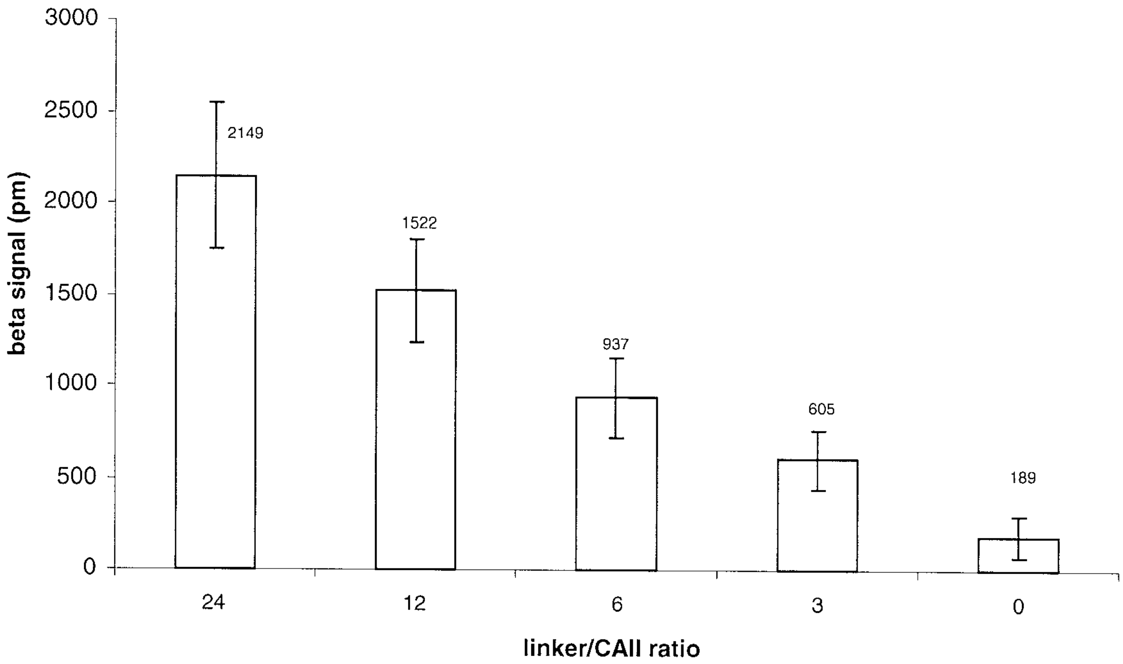
3000
2500
2000
1500
1000
500
0
beta signal (pm)
2149
1522
937
605
189
24
12
6
3
0
linker/CAII ratio

# FIG. 8

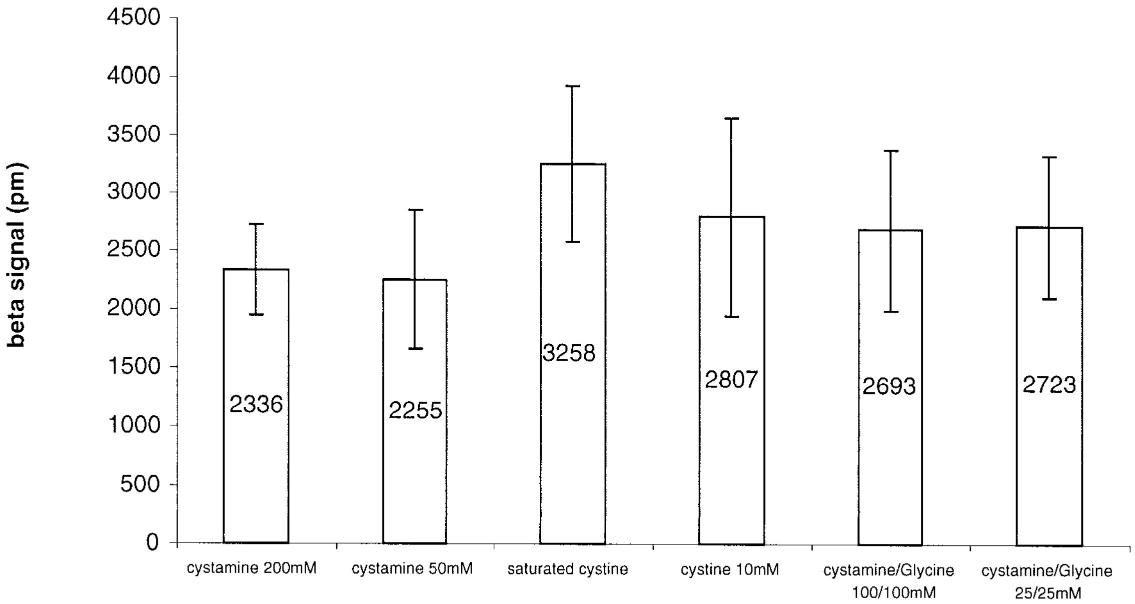
4500
4000
3500
3000
2500
2000
1500
1000
500
0
beta signal (pm)
2336
2255
3258
2807
2693
2723
cystamine 200mM
cystamine 50mM
saturated cystine
cystine 10mM
cystamine/Glycine 100/100mM
cystamine/Glycine 25/25mM

# FIG. 9

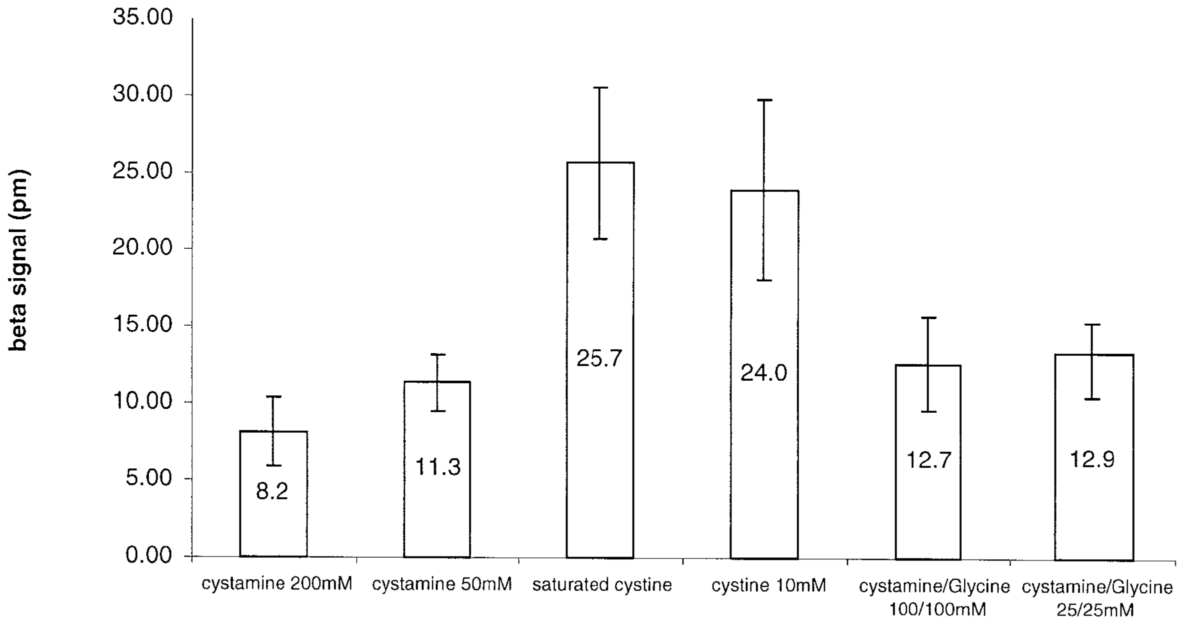

beta signal (pm)
35.00
30.00
25.00
20.00
15.00
10.00
5.00
0.00
8.2
11.3
25.7
24.0
12.7
12.9
cystamine 200mM
cystamine 50mM
saturated cystine
cystine 10mM
cystamine/Glycine 100/100mM
cystamine/Glycine 25/25mM

Europäisches Patentamt
European Patent Office
Office européen des brevets

# EUROPEAN SEARCH REPORT

Application Number
EP 09 30 5773

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2008/060268 A2 (UNIV NORTHWESTERN [US]; MIRKIN CHAD A [US]; ELGHANIAN ROBERT [US]) 22 May 2008 (2008-05-22)<br>* claims * | 1-21 | INV.<br>G01N33/543 |
| A | US 2002/115072 A1 (OKAMOTO TADASHI [JP] ET AL) 22 August 2002 (2002-08-22)<br>* claims 9,13 * | 1-21 | |
| A | US 5 834 224 A (RUGER PETRA [DE] ET AL) 10 November 1998 (1998-11-10)<br>* claims *<br>* example 7 * | 1-21 | |
| A | US 5 077 210 A (LIGLER FRANCES S [US] ET AL) 31 December 1991 (1991-12-31)<br>* claims * | 1-21 | |

TECHNICAL FIELDS SEARCHED (IPC)

G01N
C12Q

The present search report has been drawn up for all claims

1

| Place of search | Date of completion of the search | Examiner |
|---|---|---|
| The Hague | 20 May 2010 | Routledge, Brian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 09 30 5773

This annex lists the patent family members relating to the patent documents cited in the above-mentioned European search report.
The members are as contained in the European Patent Office EDP file on
The European Patent Office is in no way liable for these particulars which are merely given for the purpose of information.

20-05-2010

| Patent document cited in search report | Publication date | Patent family member(s) | Publication date |
|---|---|---|---|
| WO 2008060268 A2 | 22-05-2008 | NONE | |
| US 2002115072 A1 | 22-08-2002 | JP 4261661 B2 | 30-04-2009 |
| | | JP 2000270896 A | 03-10-2000 |
| US 5834224 A | 10-11-1998 | DE 4430023 A1 | 29-02-1996 |
| | | EP 0698787 A1 | 28-02-1996 |
| | | ES 2214488 T3 | 16-09-2004 |
| | | JP 2735817 B2 | 02-04-1998 |
| | | JP 8233773 A | 13-09-1996 |
| US 5077210 A | 31-12-1991 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 09290223 A
- US 2006013539 W
- WO 2006108183 A, Fang, Y.
- US 7175980 B
- US 6677131 B
- US 7007709 B
- US 20070141231 A
- US 61123609 B
- EP 0226470 A
- EP 08305845 A


### Non-patent literature cited in the description


- **Pompe.** Functional Films of Maleic Anhydride Copolymers under Physiological Conditions. *Macromol. Biosci.,* 2005, vol. 5, 890-895
- **Fang, Y. et al.** Resonant waveguide grating biosensor for living cell sensing. *Biophys. J.,* 2006, vol. 91, 1925-1940
- **Feiser ; Feiser.** *Reagents for Organic Synthesis,* 1967, vol. 1
- **March, J.** Advanced Organic Chemistry. John Wiley & Sons, 1992
- **House, H. O.** Modem Synthetic Reactions. W. A. Benjamin, 1972
- **Larock, R. C.** Comprehensive Organic Transformations. Wiley-VCH Publishers, 1999
- **Greene, T. W. ; Wutz, P. G. M.** Protecting Groups In Organic Synthesis. John Wiley & Sons, Inc, 1991
- *Comb. Chem. & High Throughput Screening,* 2004, vol. 7, 213-221
- *Biosensors & Bioelectronics,* 1995, vol. 10, 813-8122